# EUROPEAN PATENT APPLICATION

(11) **EP 2 653 469 A1**
(43) Date of publication of application: **23.10.2013**
(21) Application number: 11848589.5
(22) Date of filing: 16.12.2011
(51) Int. Cl.: C07D 401/14, A61K 31/4192, A61K 31/4439, A61K 31/444, A61K 31/506, A61P 1/00, A61P 1/14, A61P 5/00, A61P 9/12, A61P 25/04, A61P 25/08, A61P 25/14, A61P 25/16, A61P 25/18, A61P 25/20, A61P 25/22, A61P 25/24, A61P 25/28, A61P 25/30, A61P 29/00

(54) **PYRAZOLE DERIVATIVE**

(30) Priority: 17.12.2010 JP 2010281291
(71) Applicant: Taisho Pharmaceutical Co., Ltd., Tokyo 170-8633 (JP)
(72) Inventor: NOZAWA Dai, Tokyo 170-8633 (JP); SUZUKI Ryo, Tokyo 170-8633 (JP); FUTAMURA Aya, Tokyo 170-8633 (JP); SHIMONO Rie, Tokyo 170-8633 (JP); ABE Masahito, Tokyo 170-8633 (JP); OHTA Hiroshi, Tokyo 170-8633 (JP); ARAKI Yuko, Tokyo 170-8633 (JP)
(74) Representative: HOFFMANN EITLE
(86) International application number: PCT/JP2011/079158
(87) International publication number: WO 2012/081692

(57) **Abstract**

A compound represented by formula (IA) or a pharmaceutically acceptable salt thereof, which is useful for the treatment or prevention of diseases such sleep disorder, depression, anxiety disorder, panic disorder, schizophrenia, drug dependence, Alzheimer's disease, Parkinson's disease, Huntington's chorea, eating disorder, pain, gastrointestinal diseases, epilepsy, inflammation, immune-related diseases, endocrine-related diseases, and hypertension, and of which the action relies on an orexin (OX) receptor antagonistic activity.

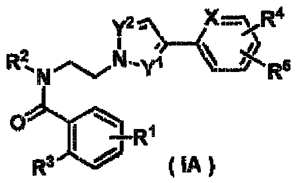

## Description

### TECHNICAL FIELD

The present invention relates to a compound having an orexin (OX) receptor antagonistic effect and a pharmaceutically acceptable salt thereof, and a therapeutic or prophylactic agent for diseases such as sleep disorder, depression, anxiety disorder, panic disorder, schizophrenia, drug dependence, Alzheimer's disease, Parkinson's disease, Huntington's chorea, eating disorder, pain, gastrointestinal diseases, epilepsy, inflammation, immune-related diseases, endocrine-related diseases, and hypertension, comprising the same as an active ingredient.

### [Background Art]

Orexin is a neuropeptide that is spliced from prepro-orexin and specifically expressed in the lateral hypothalamic area. OX-A composed of 33 amino acids and OX-B composed of 28 amino acids have been identified so far. All of these molecules play an important role in the regulation of sleep-wake patterns and the regulation of feeding.

Both OX-A and OX-B act on OX receptors. The OX receptors have been cloned so far as two subtypes: OX1 and OX2 receptors, all of which are known to be seven-transmembrane G protein-coupled receptors expressed mainly in the brain. The OX1 receptor is specifically coupled with a G protein subclass Gq, while the OX2 receptor is coupled with Gq and Gi/o (see Non Patent Literatures 1 and 2).
These OX receptors show distinct tissue distribution by their subtypes. The OX1 receptor is expressed at a high density in the locus coeruleus, which is the nucleus of origin for noradrenergic neurons, while the OX2 receptor is expressed at a high density in the tuberomammillary nucleus, which is the nucleus of origin for histamine neurons (see Non Patent Literatures 3, 4, and 5). The expression of both the OX1 receptor and the OX2 receptor is found in the raphe nucleus, which is the nucleus of origin for serotonin neurons, or in the ventral tegmental area, which is the nucleus of origin for dopamine neurons (see Non Patent Literature 3). Orexin neurons project to the monoaminergic neuronal systems in the brain stem and the hypothalamus and send excitatory influences to these neurons. In addition, the expression of the OX2 receptor is also seen in brain stem acetylcholine neurons involved in the control of REM sleep and also influences the activity of these nerve nuclei (see Non Patent Literatures 3 and 4).

In recent years, the OX1 and OX2 receptors are focused on the role of relating the sleep-wake cycle. Along with this, the usefulness of compounds having an OX receptor antagonistic effect has been studied. The intracerebroventricular administration of OX-A to rats has been confirmed, for example, to enhance locomotor activity (see Non Patent Literatures 6 and 7), enhance stereotyped behavior (see Non Patent Literature 7), and prolong arousal (see Non Patent Literature 6). The effect of shortening REM sleep by the administration of OX-A is completely antagonized by pretreatment with an OX receptor antagonist (see Non Patent Literature 8). Reportedly, the administration of orally administrable substance that antagonize OX1 and OX2 receptors at the same levels decreases the amount of locomotion, shortens sleep onset latency, and increases the amount of non-REM sleep and REM sleep (see Non Patent Literatures 9 and 10).
Patent Literature 1 discloses pyrazole derivatives as compounds having an OX receptor antagonistic effect, but does not disclose a compound having a pyrazole-ethylamide skeleton as described in the present application. By contrast, Patent Literature 2 discloses compounds having a pyrazole-ethylamide skeleton. Patent Literature 2, however, does not disclose the OX receptor antagonistic effect or the compound described in the present application.

### [Citation List]

### [Patent Literature]

[Patent Literature 1] WO2003/002559
[Patent Literature 2] WO2008/062878

### [Non Patent Literature]

[Non Patent Literature 1] Zhu Y et al., J. Pharmacol. Sci., 92, 259-266, 2003.
[Non Patent Literature 2] Zeitzer JM et al., Trends Pharmacol. Sci., 27, 368-374, 2006.
[Non Patent Literature 3] Marcus JN et al., J. Comp. Neurol, 435, 6-25, 2001.
[Non Patent Literature 4] Trivedi JP et al., FEBS Lett, 438, 71-75, 1998.
[Non Patent Literature 5] Yamanaka A et al., Biochem. Biophys. Res. Commun., 290, 1237-1245, 2002.
[Non Patent Literature 6] Hagan JJ et al., Proc. Natl. Acad. Sci. USA, 96, 10911-10916, 1999.
[Non Patent Literature 7] Nakamura T et al., Brain Res., 873, 181-187, 2000.
[Non Patent Literature 8] Smith MI et al., Neurosci. Lett., 341, 256-258, 2003.
[Non Patent Literature 9] Brisbare-Roch C et al., Nat. Med., 13, 150-155, 2007.
[Non Patent Literature 10] Cox CD et al., J. Med. Chem., 53, 5320-5332, 2010.

### [Summary of Invention]

### [Technical Problem]

An object of the present invention is to discover novel compounds with antagonistic actions on OX receptor and to provide a therapeutic or prophylactic agent for diseases such as sleep disorder, depression, anxiety disorder, panic disorder, schizophrenia, drug dependence, Alzheimer's disease, Parkinson's disease, Huntington's chorea, eating disorder, pain, gastrointestinal disease, epilepsy, inflammation, immune-related diseases, endocrine-related diseases, and hypertension. More specifically, an object of the present invention is to provide a novel compound that has an excellent OX receptor antagonistic effect and also exhibits excellent pharmacokinetics and safety.

### [Solution to Problem]

The present inventors have conducted diligent studies on compounds with a novel skeleton having an antagonistic effect on orexin receptors and consequently completed the present invention by finding that a certain type of pyrazole derivative represented by formula shown below has an excellent OX receptor antagonistic effect.
Hereinafter, the present invention will be described in detail. The aspect of the present invention (hereinafter, preferred to as the "compound of the present invention") is as follows:
(1) A pyrazole derivative represented by formula (IA) or a pharmaceutically acceptable salt thereof: 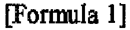 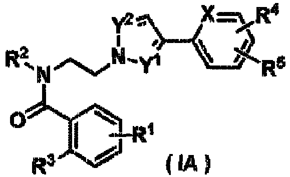 wherein
   X represents a nitrogen atom or a formula CH;
   any one of Y¹ and Y² represents a nitrogen atom, and the other of Y¹ and Y² represents a formula CH;
   R¹ represents a hydrogen atom, a halogen atom, a C₁₋₆ alkyl group, or a C₁₋₆ alkoxy group;
   R² represents a hydrogen atom, a C₁₋₆ alkyl group, wherein the C₁₋₆ alkyl group may be substituted with 1 to 3 substituents selected from Substituent group 1., a C₃₋₆ cycloalkyl group, or a 4- to 6-membered cyclic ether group,
   wherein. Substituent group 1 is a group consisting of a halogen atom, a C₃₋₆ cycloalkyl group, and a C₁₋₆ alkoxy group;
   R³ represents a triazolyl group, a pyridyl group, or a pyrimidyl group,
   wherein the triazolyl group, the pyridyl group, or the pyrimidyl group may be substituted with 1 to 3 halogen atoms; and
   R⁴ and R⁵ are the same or different and each represent a hydrogen atom, a halogen atom, or a C₁₋₆ alkyl group, wherein the C₁₋₆ alkyl group may be substituted with 1 to 3 halogen atoms.
(2) The pyrazole derivative or a pharmaceutically acceptable salt thereof according to (1), wherein R⁵ is a hydrogen atom.
(3) A pyrazole derivative represented by formula (I) or a pharmaceutically acceptable salt thereof: 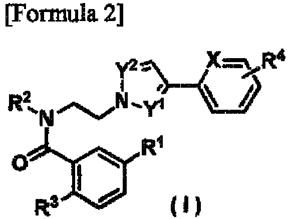 wherein
   X represents a nitrogen atom or a formula CH;
   any one of Y¹ and Y² represents a nitrogen atom, and the other of Y¹ and Y² represents a formula CH;
   R¹ represents a hydrogen atom, a halogen atom, or a C₁₋₆ alkyl group;
   R² represents a hydrogen atom, a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group,
   wherein Substituent group 1 is a group consisting of a halogen atom, a C₃₋₆ cycloalkyl group, and a C₁₋₆ alkoxy group;
   R³ represents a triazolyl group, a pyridyl group, or a pyrimidyl group,
   wherein the triazolyl group, the pyridyl group, or the pyrimidyl group may be substituted with 1 to 3 halogen atoms; and
   R⁴ represents a hydrogen atom, a halogen atom, or a C₁₋₆ alkyl group.
(4) The pyrazole derivative or a pharmaceutically acceptable salt thereof according to any one of (1) to (3), wherein R² is a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group.
(5) The pyrazole derivative or a pharmaceutically acceptable salt thereof according to any one of (1) to (4), wherein R² is a methyl group or an ethyl group.
(6) The pyrazole derivative or a pharmaceutically acceptable salt thereof according to any one of (1) to (5), wherein
   X is a nitrogen atom, and
   R³ is a triazolyl group or a pyrimidyl group.
(7) Any one or a mixture of two or more selected from the following group of the compounds and pharmaceutically acceptable salts thereof according to (1):
   N-{2-[4-(5-fluoropyridin-2-yl)-1H-pyrazol-1-yl]ethyl}-5-methyl-2-(2H-1,2,3-triazol-2-yl)benzamide,
   N-{2-[4-(5-fluoropyridin-2-yl)-1H-pyrazol-1-yl]ethyl}-N,5-dimethyl-2-(2H-1,2,3-triazol-2-yl)benzamide,
   N-{2-[4-(4-fluorophenyl)-1H-pyraol-1-yl]ethyl}-5-methyl-2-(2H-1,2,3-triazol-2-yl)benzamide,
   N-{2-[4-(4-fluorophenyl)-1H-pyrazol-1-yl]ethyl}-N,5-dimethyl-2-(2H-1,2,3-triazol-2-yl)benzamide,
   N-ethyl-N-{2-[4-(5-fluoropyridin-2-yl)-1H-Pyrazal-1-yl]ethyl}-5-methyl-2-{2H-1,2,3-triazal-2-yl)benzamide,
   N-(cyclopropylmethyl)-N-{2-[4-(5-fluoropyridin-2-yl)-1H-pyrazol-1-yl]ethyl}-5-methyl-2-(2H-1,2,3-triazol-2-yl)benzamide,
   N-(cyclopropylmethyl)-N-{2-[4-(4-fluorophenyl)-1H-pyrazol-1-yl]ethyl}-5-methyl-2-(2H-1,2,3-triazol-2-yl)benzamide,
   N-ethyl-N-{2-[4-(4-fluorophenyl)-1H-pyrazo]-1-yl]ethyl}-5-methyl-2-(2H-1,2,3-triazol-2-yl)benzamide,
   N-ethy]-N-{2-[3-(4-fluorophenyl)-1H-pyrazol-1-yl]ethyl}-5-methyl-2-(2H-1,2,3-triazol-2-yl)benzamide,
   N-ethyl-5-methyl-N-{2-[3-(pyridin-2-yl)-1H-pyrazol-1-yl]ethyl}-2-(2H-1,2,3-triazol-2-yl)benzamide,
   N-{2-[4-{5-chloropyridin-2-yl)-1H-pyrazol-1-yl]ethyl}-N-ethyl-5-methyl-2-(2H-1,2,3-triazol-2-yl)benzamide,
   N-ethyl-5-methyl-N-{2-[4-(pyridin-2-yl)-1H-pyrazol-1-yl]ethyl}-2-(2H-1,2,3-triazol-2-yl)benzamide,
   N-ethyl-N-{2-[4-(6-methylpyridin-2-yl)-1H-pyrazol-1-yl]ethyl}-5-methyl-2-(2H-1,2,3-triazol-2-yl)benzami.de,
   N-cyclopropyl-N-{2-[4-(5-fluoropyridin-2-yl)-1H-pyrazol-1-yl]ethyl}-5-methyl-2-(2H-1,2,3-triazol-2-yl)benzamide,
   N-{2-[4-(5-fluoropyridin-2-yl)-1H-pyrazol-1-yl]ethyl}-5-methyl-N-(propan-2-yl)-2-(2H-1,2,3-triazol-2-yl)benzamide,
   N-ethyl-N-{2-[4-(5-fluoropyridin-2-yl)-1H-pyrazol-yl]ethyl}-5-methyl-2-(pyrimidin-2-yl)benzamide,
   N-{2-[3-(5-fluoropyridin-2-yl)-1H-Pyrazol-1-yl]ethyl}-5-methyl-2-(2H-1,2,3-triazol-2-yl)benzamide,
   N-{2-[3-(5-fluoropyridin-2-yl)-1H-pyrazol-1-yl]ethyl}-N,5-dimethyl-2-(2H-1,2,3-triazol-2-yl)benzamide,
   N-(cyclopropylmethyl)-N-{2-[3-(5-fluoropyridin-2-yl)-1H-pyrazol-1-yl]ethyl}-5-methyl-2-(2H-1,2,3-triazol-2-yl)benzamide,
   N-ethyl-N-{2-[3-(5-fluoropyridin-2-yl)-1H-pyrazol-1-yl]ethyl}-5-methyl-2-(2H-1,2,3-triazol-2-yl)benzamide,
   N-{2-[3-(4-fluorophenyl)-1H-pyrazol-1-yl]ethyl}-5-methyl-2-(2H-1,2,3-triazol-2-yl)benzamide,
   N-{2-[3-(4-fluorophenyl)-1H-pyrazol-1-yl]ethyl}-N,5-dimethyl-2-(2H-1,2,3-triazol-2-yl)benzamide,
   N-{2-[3-(5-fluoropyridin-2-yl)-1H-pyrazol-1-yl]ethyl}-5-methyl-N-(propan-2-yl)-2-(2H-1,2,3-triazol-2-yl)benzamide,
   N-(2,2-difluoroethyl)-N-{2-[3-(5-fluoropyridin-2-yl)-1H-pyrazol-1-yl]ethyl}-5-methyl-2-(2H-1,2,3-triazol-2-yl)benzamide,
   5-fluoro-N-{2-[3-(5-fluoropyridin-2-yl)-1H-pyrazol-1-yl]ethyl}-2-(2H-1,2,3-triazol-2-yl)benzamide,
   N-methyl-N-{2-[3-(5-fluoropyridin-2-yl)-1H-pyrazol-1-yl]ethyl}-5-fluoro-2-(2H-1,2,3-triazol-2-yl)benzamide,
   N-ethyl-N-{2-[3-(5-fluoropyridin-2-yl)-1H-pyrazol-1-yl]ethyl}-5-fluoro-2-(2H-1,2,3-triazol-2-yl)benzamide,
   N-{2-[3-(5-fluoropyridin-2-yl)-1H-pyrazol-1-yl]ethyl}-5-methyl-2-(pyrimidin-2-yl)benzamide,
   N-ethyl-N-{2-[3-(5-fluoropyridin-2-yl)-1H-pyrazol-1-yl]ethyl}-5-methyl-2-(pyrimidin-2-yl)benzamide,
   N-{2-[3-(5-fluoropyridin-2-yl)-1H-Pyrazol-1-yl]ethyl}-5-methyl-N-(2-fluoroethyl)-2-(2H-1,2,3-triazol-2-yl)benzamide,
   N-(cyclopropylmethyl)-N-{2-[3-(4-fluorophenyl)-1H-pyrazol-1-yl]ethyl}-5-methyl-2-(2H-1,2,3-triazol-2-yl)benzamide,
   N-(cyclopropylmethyl)-5-methyl-N-[2-(3-phenyl-1H-pyrazol-1-yl)ethyl]-2-(2H-1,2,3-triazol-2-yl)benzamide,
   N-cyclopropyl-N-{2-[3-(5-fluoropyridin-2-yl)-1H-pyrazol-1-yl]ethyl}-5-methyl-2-(2H-1,2,3-triazol-2-yl)benzamide,
   N-{2-[3-(5-fluoropyridin-2-yl)-1H-pyrazol-1-yl]ethyl}-5-methyl-N-(oxetan-3-yl)-2-(2H-1,2,3-triazol-2-yl)benzamide,
   N-ethyl-N-{2-[3-(5-fluoropyridin-2-yl)-1H-pyrazol-1-yl]ethyl}-2-(2H-1,2,3-triazol-2-yl)benzamide,
   5-chloro-N-ethyl-N-{2-[3-(5-fluoropyridin-2-yl)-1H-pyrazol-1-yl]ethyl}-2-(2H-1,2,3-triazol-2-yl)benzamide,
   N-ethyl-N-{2-[3-(5-fluoropyridin-2-yl)-1H-pyrazol-1-yl]ethyl}-2-(pyrimidin-2-yl)benzamide,
   N-ethyl-N-{2-[3-(5-fluoropyridin-2-yl)-1H-pyrazol-1-yl]ethyl}-5-methyl-2-(pyridin-2-yl)benzamide,
   N-ethyl-N-{2-[3-(5-fluoropyridin-2-yl)-1H-pyrazol-1-yl]ethyl}-5-methyl-2-(pyridin-3-yl)benzamide,
   N-ethyl-N-{2-[4-(5-fluoropyridin-2-yl)-1H-pyrazol-1-yl]ethyl}-2-(pyrimidin-2-yl)benzamide, 5-methyl-N-{2-[3-(6-methylpyridin-2-yl)-1H-pyrazol-1-yl]ethyl}-2-(2H-1,2,3-triazol-2-yl)benzamide,
   N-ethyl-5-methyl-N-{2-[3-(6-methylpyridin-2-yl)-1H-pyrazol-1-yl]ethyl}-2-(2H-1,2,3-triazol-2-yl)benzamide,
   5-fluoro-N-{2-[3-(5-fluoropyridin-2-yl)-1H-pyrazol-1-yl]ethyl}-2-(pyrimidin-2-yl)benzamide,
   N-ethyl-5-fluoro-N-{2-[3-(5-fluoropyridin-2-yl)-1H-pyrazol-1-yl]ethyl}-2-(pyrimidin-2-yl)benzamide,
   5-chloro-N-{2-[3-(5-fluoropyridin-2-yl)-1H-pyrazol-1-yl]ethyl}-2-(pyrimidin-2-yl)benzamide,
   N-ethyl-5-chloro-N-{2-[3-(5-fluoropyridin-2-yl)-1H-pyrazol-1-yl]ethyl}-2-(pyrimidin-2-yl)benzamide,
   N-ethyl-4-fluoro-N-{2-[3-(5-fluoropyridin-2-yl)-1H-pyrazol-1-yl]ethyl}-2-(2H-1,2,3-triazol-2-yl)benzamide,
   N-ethyl-2-fluoro-N-{2-[3-(5-fluoropyridin-2-yl)-1H-pyrazol-1-yl]ethyl}-6-(2H-1,2,3-triazol-2-yl)benzamide,
   N-ethyl-2-fluoro-N-{2-[3-(5-fluoropyridin-2-yl)-1H-pyrazol-1-yl]ethyl}-6-(pyrimidin-2-yl}benzamide,
   N-ethyl-N-{2-[3-(5-fluoropyridin-2-yl)-1H-pyrazol-1-yl]ethyl}-4-methyl-2-(2H-1,2,3-triazol-2-yl)benzamide,
   N-ethyl-3-fluoro-N-{2-[3-(5-fluoropyridin-2-yl)-1H-pyrazol-1-yl]ethyl}-2-(2H-1,2,3-triazol-2-yl)benzamide,
   N-ethyl-4-fluoro-N-{2-[3-(5-fluoropyridin-2-yl)-1H-pyrazol-1-yl]ethyl}-2-(pyrimidin-2-yl)benzamide,
   N-ethyl-N-{2-[3-(5-fluoropyridin-2-yl)-1H-pyrazol-1-yl]ethyl}-5-methoxy-2-(2H-1,2,3-triazol-2-yl)benzamide,
   N-ethyl-5-methyl-2-(2H-1,2,3-triazol-2-yl)-N-(2-{3-[5-(trifluoromethyl)pyridin-2-yl]-1H-pyrazol-1-yl}ethyl)benzamide,
   N-ethyl-5-methyl-2-(2H-1,2,3-triazol-2-yl)-N-(2-{3-[6-(trifluoromethyl)pyridin-2-yl]-1H-pyrazol-1-yl}ethyl)benzamide,
   N-{2-[3-(3,4-difluorophenyl)-1H-pyrazol-1-yl]ethyl}-N-ethyl-5-fluoro-2-(pyrimidin-2-yl)benzamide,
   N-ethyl-5-fluoro-N-{2-[3-{4-fluorophenyl)-1H-pyrazol-1-yl]ethyl}-2-(pyrimidin-2-yl)benzamide,
   5-chloro-N-ethyl-N-{2-[4-(5-fluoropyridin-2-yl)-1H-pyrazol-1-yl]ethyl}-2-(2H-1,2,3-triazol-2-yl)benzamide,
   N-ethyl-5-fluoro-N-{2-[4-(5-fluoropyridin-2-yl)-1H-pyrazol-1-yl]ethyl}-2-(2H-1,2,3-triazol-2-yl)benzamide,
   N-ethyl-5-fluoro-2-(pyrimidin-2-yl)-N-(2-{3-[6-(trifluoromethyl)pyridin-2-yl]-1H-pyrazol-1-yl}ethyl)benzamide,
   N-ethyl-(pyrimidin-2-yl)-N-(2-{3-[6-(trifluoromethyl)pyridin-2-yl]-1H-pyrazol-1-yl}ethyl)benzamide,
   N-{2-[3-(3,4-difluorophenyl)-1H-pyrazol-1-yl]ethyl}-N-ethyl-2-(pyrimidin-2-yl)benzamide,
   N-ethyl-5-methyl-2-(2H-1,2,3-triazol-2-yl)-N-(2-{3-[4-(trifluoromethyl)pyridin-2-yl]-1H-pyrazol-1-yl}ethyl)benzamide,
   N-ethyl-5-fluoro-2-(pyrimidin-2-yl)-N-(2-{3-[4-(trifluoromethyl)pyridin-2-yl]-1H-pyrazol-1-yl}ethyl)benzamide.
(8) A pharmaceutical composition comprising a pyrazole derivative or a pharmaceutically acceptable salt thereof according to any one of (1) to (7) as an active ingredient.
(9) A therapeutic or prophylactic agent for a disease selected from sleep disorder, depression, anxiety disorder, panic disorder, schizophrenia, drug dependence, Alzheimer's disease, Parkinson's disease, Huntington's chorea, eating disorder, pain, gastrointestinal disease, epilepsy, inflammation, immune-related diseases, endocrine-related diseases, and hypertension, comprising a pyrazole derivative or a pharmaceutically acceptable salt thereof according to any one of (1) to (7) as an active ingredient.

### [Advantageous Effects of Invention]

The pyrazole derivative of the present invention has been shown to exhibit affinity for OX receptors and also exhibit an antagonistic effect on the stimulation of the receptors by physiological ligands.

### [Description of Embodiments]

The terms used in the present specification have the following meanings:
The "halogen atom" refers to a fluorine atom, a chlorine atom, a bromine atom, or an iodine atom.
The "C₁₋₆ alkyl group" means a linear or branched alkyl group having 1 to 6 carbon atoms. Examples thereof include methyl, ethyl, n-propyl, isopropyl, n-butyl, isobutyl, sec-butyl, tert-butyl, n-pentyl, isopentyl, neopentyl, tert-pentyl, 1-ethylpropyl, n-hexyl, isohexyl, and neohexyl groups.
The "C₃₋₆ cycloalkyl group" refers to a cyclopropyl, cyclobutyl, cyclopentyl, or cyclohexyl group.
The "4- to 6-membered cyclic ether group" refers to an oxetanyl, tetrahydrofuranyl, or tetrahydropyranyl group.
The "C₁₋₆ alkoxy group" means a linear or branched alkoxy group having 1 to 6 carbon atoms. Examples thereof include methoxy, ethoxy, n-propoxy, isopropoxy, n-butoxy, isobutoxy, sec-butoxy, tert-butoxy, n-pentyloxy, isopentyloxy, neopentyloxy, tert-pentyloxy, 1-ethylpropoxy, and n-hexyloxy groups.

The "pharmaceutically acceptable salt" described in the present specification means an acid-addition salt that can be accepted for pharmaceuticals. The salt with the acid used includes: salts with inorganic acids such as sulfuric acid, hydrochloric acid, hydrobromic acid, phosphoric acid., and nitric acid; and salts with organic acids such as acetic acid, benzoic acid, oxalic acid, lactic acid, malic acid, tartaric acid, fumaric acid, maleic acid, citric acid, malonic acid, mandelic acid, gluconic acid, galactaric acid, glucoheptonic acid, glycolic acid, glutamic acid, methanesulfonic acid, ethanesulfonic acid, benzenesulfonic acid, p-toluenesulfonic acid, camphorsulfonic acid, and naphthalene-2-sulfonic acid. The salt can be converted from a free form by a conventional method.

Preferred aspects of the compound of the present invention will be shown below.
The compound wherein X is a nitrogen atom is preferred.
The compound wherein the halogen atom is a fluorine atom, a chlorine atom, or a bromine atom is preferred, and the compound wherein the halogen atom is a fluorine atom or a chlorine atom is more preferred.
The compound wherein R² is a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group is preferred, and the compound wherein R² is a methyl group or an ethyl group is more preferred.
The compound wherein R³ is a triazolyl group or a pyrimidyl group is preferred.
The compound wherein R⁵ is a hydrogen atom or a halogen atom is preferred, and the compound wherein R⁵ is a hydrogen atom is more preferred.
When the compound of the present invention forms a hydrate or a solvate, the hydrate and the solvate are also included in the scope of the present invention. Likewise, a pharmaceutically acceptable salt of such a hydrate or solvate of the compound of the present invention is also included in the scope of the present invention.

The compound of the present invention includes all of enantiomers, diastereomers, equilibrium compounds, mixtures thereof at arbitrary ratios, racemates, etc.
The compound according to the present invention also includes compounds containing one or more hydrogen atoms, carbon atoms, nitrogen atoms, oxygen atoms, or fluorine atoms substituted with radioisotopes or stable isotopes. Such labeled compounds are useful in metabolic or pharmacokinetic study and also useful, for example, as a ligand for the receptor, in biological analysis, etc.
The compound according to the present invention can be administered orally or parenterally. Its dosage form is any of tablets, capsules, granules, powders, dusts, troches, ointments, creams, skin patches, emulsions, suspensions, suppositories, injections, and the like. All of these dosage forms can be produced by a pharmaceutical technique routinely used (e.g., methods specified by the Japanese Pharmacopoeia, 15th Edition). The dosage form can be selected appropriately according to the symptom, age, body weight, and therapeutic purpose of the patient.
The preparations can be produced by mixing a composition containing the compound of the present invention with pharmacologically acceptable carriers, i.e., an excipient (e.g., crystalline cellulose, starch, lactose, and mannitol), a binder (e.g., hydroxypropylcellulose and polyvinylpyrrolidone), a lubricant (e.g., magnesium stearate and talc), a disintegrant (e.g., carboxymethylcellulose calcium), and other various pharmacologically acceptable additives.
The compound of the present invention can be administered orally or parenterally once a day or several times a day at each dose of 0.001 to 500 mg to an adult patient. This dose can be increased or decreased appropriately according to the type of disease to be treated, the age, body weight, and symptom of the patient, etc.

Typical methods for producing compound (I) of the present invention are shown in Schemes A to I below. These methods for producing the compound of the present invention are provided merely for illustrative purposes and do not limit the present invention. In the examples of the production methods shown below, each compound may form a salt that does not hinder the reaction.

### Scheme A

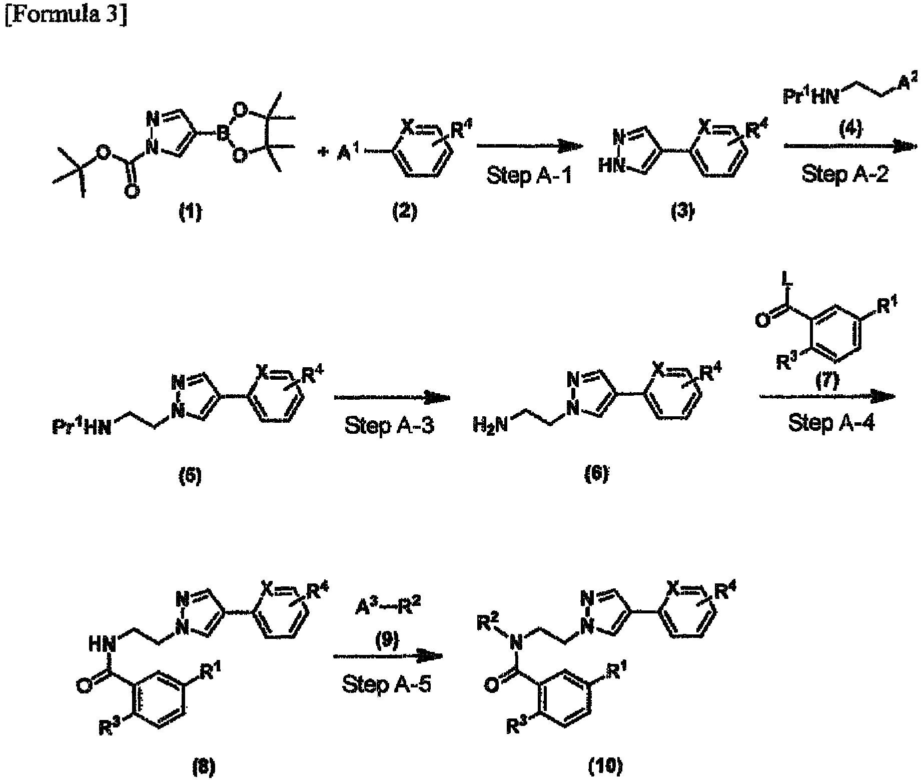

wherein X, R¹, R², R³, and R⁴ are as defined above; A¹, A², and A³ each represent a halogen atom, a methanesulfonyloxy group, a p-toluenesulfonyloxy group, or a trifluoromethanesulfonyloxy group; L represents a hydroxy group or a halogen atom; and Pr¹ represents a protective group for amino groups routinely used, described in "Protective Groups in Organic Chemistry" by J.F.W. McOmie, and "Protective Groups in Organic Synthesis" by T.W. Greene and P.G.M. Wuts.

Step A-1: A compound represented by formula (3) can be obtained by the reaction between a boronic acid derivative represented by formula (1) and a compound represented by formula (2) under conditions of Suzuki-Miyaura coupling reaction. The comprehensive overview of the Suzuki-Miyaura coupling reaction may be found in Angew. Chem. Int. Ed. 2001, 40, 4544.

Step A-2: A compound represented by formula (5) can be obtained by the reaction between the compound represented by formula (3) and a compound represented by formula (4). The reaction in Step A-2 proceeds under a temperature condition of around -80°C to around the boiling point of a solvent in the presence of an inorganic base such as sodium hydride, sodium hydroxide, sodium carbonate, potassium carbonate, or cesium carbonate or an organic base such as lower alkoxide of an alkali metal or alkaline earth metal (e.g., sodium ethoxide and potassium tert-butoxide), triethylamine, or diisopropylethylamine in a solvent such as N,N-dimethylformamide, dimethyl sulfoxide, acetonitrile, tetrahydrofuran, ethanol, water, or chloroform or in a mixed solvent thereof

Step A-3: In the case where Pr¹ in the compound represented by formula (5) is a group that is deprotectable by an acid, such as a tert-butoxycarbonyl group, a compound represented by formula (6) can be obtained by the reaction between the compound represented by formula (5) and an acid such as hydrochloric acid, sulfuric acid, trifluoroacetic acid, p-toluenesulfonic acid, or methanesulfonic acid. In the case where Pr¹ in the compound represented by formula (5) is a group that is deprotectable by hydrogenolysis, such as a benzyloxycarbonyl group, the group can be deprotected through hydrogenolysis reaction using a metal catalyst such as palladium. The comprehensive overview of the reaction in Step A-3 may be found in "Protective Groups in Organic Chemistry" by J.F.W McOmie, and "Protective Groups in Organic Synthesis" by T.W Greene and P.G.M. Wuts.

Step A-4: A. compound represented by formula (8) can be obtained by the reaction between the compound represented by formula (6) and a compound represented by formula (7). In the case where L in the compound represented by formula (7) is a hydroxy group, examples of the amidation reaction of Step A-4 include a method using a dehydration-condensation agent. Examples of the dehydration-condensation agent include 1-ethyl-3-(3-dimethylaminopropyl)carbodiimide hydrochloride, propylphosphonic acid anhydride (cyclic trimer), dicyclohexylcarbodiimide, diphenylphosphonyl azide, and carbonyldiimidazole. If necessary, an activator such as 1-hydroxybenzotriazole or hydroxysuccinimide can be used. Examples of the reaction solvent include N,N-dimethylformamide, tetrahydrofuran, dichloromethane, chloroform, toluene, and ethyl acetate, and mixed solvents thereof This reaction can be performed using a base. Examples of the base include: organic amines such as triethylamine and diisopropylethylamine; and inorganic bases such as potassium carbonate. The reaction can be performed at approximately -80°C to around the boiling point of the reaction solvent. In the case where L in the compound represented by formula (7) is a halogen atom, the reaction in Step A-4 proceeds under a temperature condition of around -80°C to around the boiling point of a solvent in the presence of an inorganic base such as sodium hydride, sodium hydroxide, sodium carbonate, potassium carbonate, or cesium carbonate or an organic base such as lower alkoxide of a metal (e.g., sodium ethoxide and potassium tert-butoxide), triethylamine, or diisopropylethylamine in a solvent such as N,N-dimethylformamide, dimethyl sulfoxide, acetonitrile, tetrahydrofuran, ethanol, water, or chloroform or in a mixed solvent thereof.

Step A-5: A compound represented by formula (10) can be obtained by the reaction between the compound represented by formula (8) and a compound represented by formula. (9). The reaction in Step A-5 proceeds under a temperature condition of around -80°C to around the boiling point of a solvent in the presence of an inorganic base such as sodium hydride, sodium hydroxide, sodium carbonate, potassium carbonate, or cesium carbonate or an organic base such as lower alkoxide of a metal (e.g., sodium ethoxide and potassium tert-butoxide), triethylamine, or diisopropylethylamine in a solvent such as N,N'-dimethylformamide, dimethyl sulfoxide, acetonitrile, tetrahydrofuran, ethanol, water, or chloroform or in a mixed solvent thereof.

### Scheme B

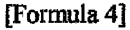

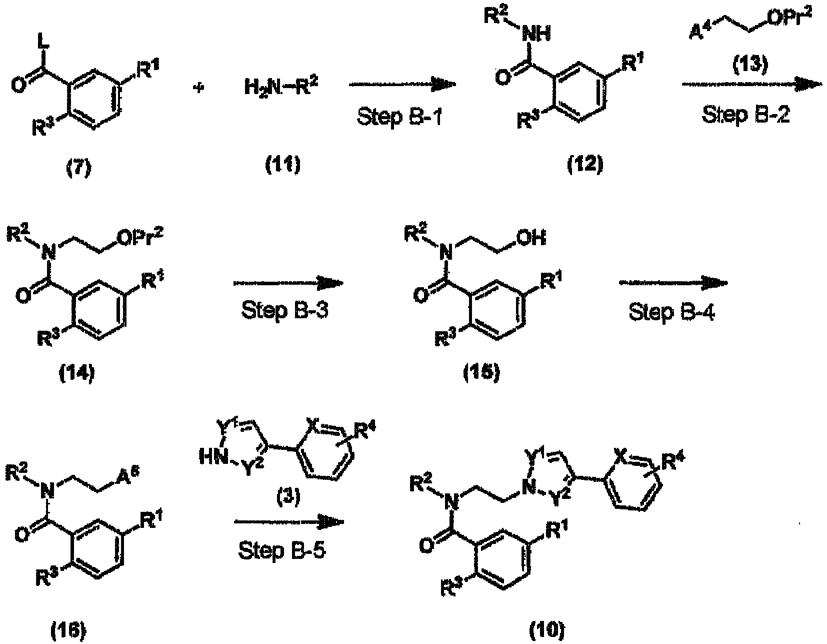

wherein X, Y¹, Y², R¹, R², R³, R⁴, and L are as defined above; A⁴ and A⁵ each represent a halogen atom, a methanesulfonyloxy group, a p-toluenesulfonyloxy group, or a trifluoromethanesulfonyloxy group; and Pr² represents a protective group for hydroxy groups routinely used, described in "Protective Groups in Organic Chemistry" by J.F.W. McOmie, and "Protective Groups in Organic Synthesis" by T.W Greene and P.G.M. Wuts.

Step B-1: A compound represented by formula (12) can be obtained by the reaction between a compound represented by formula (7) and an amine derivative represented by formula (11) according to the same reaction conditions as in Step A-4.

Step B-2: A compound represented by formula (14) can be obtained by the reaction between the compound represented by formula (12) and a compound represented by formula (13) according to the same reaction conditions as in Step A-5.

Step B-3: A compound represented by formula (15) is obtained by the removal of the protective group for the hydroxy group in the compound represented by formula (14). The comprehensive overview of the reaction in Step B-3 may be found in "Protective Groups in Organic Chemistry" by J.F.W. McOmie, and "Protective Groups in Organic Synthesis" by T.W. Greene and P.G.M. Wuts. In the case where Pr² in the compound represented by formula (14) is a silicon-containing protective group such as a tert-butyldimethylsilyl group, the compound represented by formula (15) can be obtained by the reaction between the compound represented by formula (14) and an acid (e.g., hydrochloric acid and trifluoroacetic acid) or a fluoride (e.g., tetrabutylammonium fluoride and hydrogen fluoride).

Step B-4: A compound represented by formula (16) can be obtained by the conversion of the hydroxy group in the compound represented by formula (15) to a usual leaving group. Examples of the reaction in Step B-4 include chlorination, bromination, iodization, methanesulfonylation, and p-toluenesulfonylation. Examples of the chlorination reaction include a method involving converting the hydroxy group to a leaving group using methanesulfonyl chloride or the like, and then substituting the leaving group by a chlorine atom. Further examples thereof include a method using carbon tetrachloride and triphenylphosphine, and a method using thionyl chloride or phosphorus oxychloride. In this reaction, a chloride such as sodium chloride or potassium chloride may be added. Examples of the bromination reaction include a method using carbon tetrabromide and triphenylphosphine. Examples of the iodization reaction include a method using iodine, triphenylphosphine, and imidazole. The methanesulfonylatian and the p-toluenesulfonylation can be performed using, for example, methanesulfonyl chloride and p-toluenesulfonyl chloride, respectively. In these reactions, an appropriate base may be added. Examples of the base that may be added include: organic bases such as triethylamine and diisopropylethylamine; and inorganic bases such as potassium carbonate. Examples of the reaction solvent include solvents such as N,N-dimethylformamide, dimethyl sulfoxide, acetonitrile, tetrahydrofuran, water, carbon tetrachloride, chloroform, dichloroethane, and 1,2-dichloroethane, and mixed solvents thereof. The reaction can be performed under a temperature condition of around -80°C to around the boiling point of the solvent.

Step B-5: A compound represented by formula (10) can be obtained by the reaction between the compound represented by formula (16) and a pyrazole derivative represented by formula (3) according to the same reaction conditions as in Step A-2.

### Scheme C

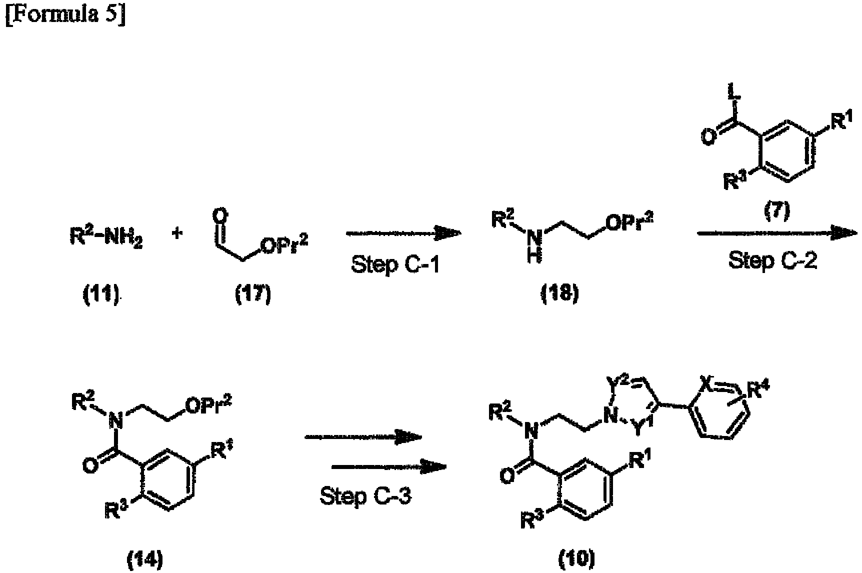

wherein X, Y¹, Y², R¹, R², R³, R⁴, L, and Pr² are as defined above.
Step C-1: A compound represented by formula (18) can be obtained by the reaction between an amine derivative represented by formula (11) and an aldehyde derivative represented by formula (17) under conditions of reductive amination reaction. The conditions of reductive animation reaction in Step C-1 involve reacting the amine derivative and the aldehyde derivative in the presence or absence of an acid and a base by the addition of a reducing agent. For example, a catalytic reduction method based on hydrogenation using a catalyst such as palladium-carbon, platinum, Raney nickel, or rhodium-alumina may be used as a reduction method. Examples of the acid include acetic acid and hydrochloric acid. Examples of the base include triethylamine. Examples of the reducing agent include sodium borohydride, sodium triacetoxyborohydride, and sodium cyanoborohydride. Examples of the reaction solvent include methanol, ethanol, diethyl ether, tetrahydrofuran, 1,4-dioxane, chloroform, dichloromethane, N,N-dimethylformamide, acetonitrile, and water, and mixed solvents thereof. The reaction can be performed under a temperature condition of around -80°C to around the boiling point of the solvent.

Step C-2: A compound represented by formula (14) can be obtained by the reaction between the amine derivative represented by formula (18) and a compound represented by formula (7) according to the same reaction conditions as in Step A-4.
Step C-3: A compound represented by formula (10) can be obtained from the compound represented by formula (14) according to the same reaction conditions as in Steps B-3, B-4, and B-5.

### Scheme D

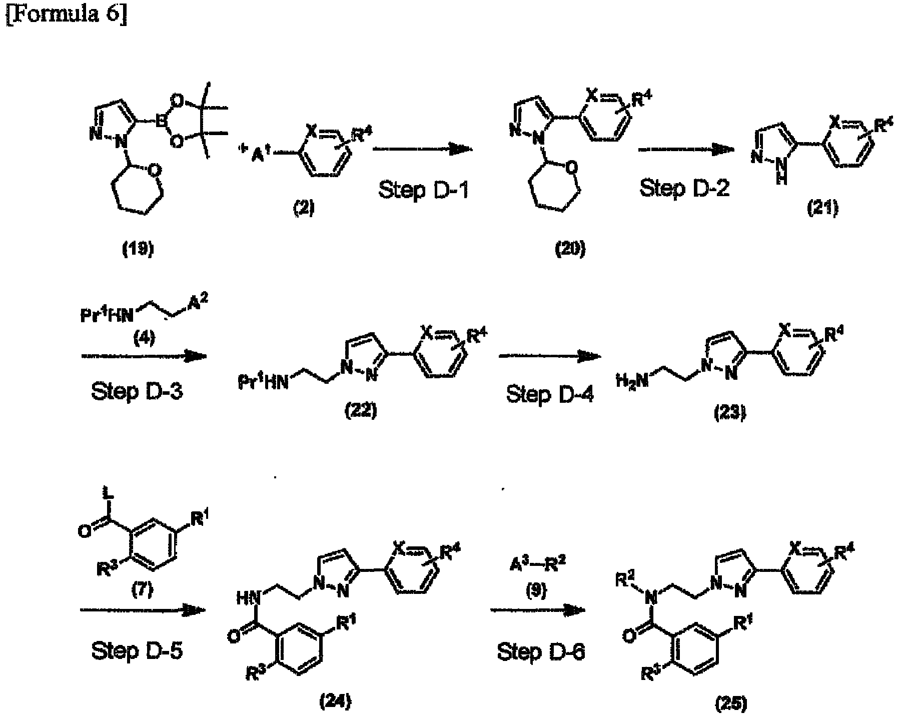

wherein X, R¹, R², R³, R⁴, A¹, A², A³, L, and Pr¹ are as defined above.
Step D-1: A compound represented by formula (20) can be obtained by the reaction between a boronic acid derivative represented by formula (19) and a compound represented by formula (2) under the same conditions of Suzuki-Miyaura coupling reaction as in Step A-1.
Step D-2: A compound represented by formula (21) can be obtained by the reaction between the compound represented by formula (20) and an acid such as hydrochloric acid or trifluoroacetic acid. Examples of the reaction solvent include solvents such as methanol, ethanol, tetrahydrofuran, 1,4-dioxane, water, ethyl acetate, and chloroform, and mixed solvents thereof. The reaction can be performed under a temperature condition of around -80°C to around the boiling point of the solvent.

Step D-3: A compound represented by formula (22) can be obtained by the reaction between the compound represented by formula (21) and a compound represented by formula (4) according to the same reaction conditions as in Step A-2.
Step D-4: A compound represented by formula (23) can be obtained by reaction from the compound represented by formula (22) according to the same reaction conditions as in Step A-3. Step D-5: A compound represented by formula (24) can be obtained by the reaction between the amine derivative represented by formula (23) and a compound represented by formula (7) according to the same reaction conditions as in Step A-4.
Step D-6: A compound represented by formula (25) can be obtained by the reaction between the compound represented by formula (24) and a compound represented by formula (9) according to the same reaction conditions as in Step A-5.

### Scheme E

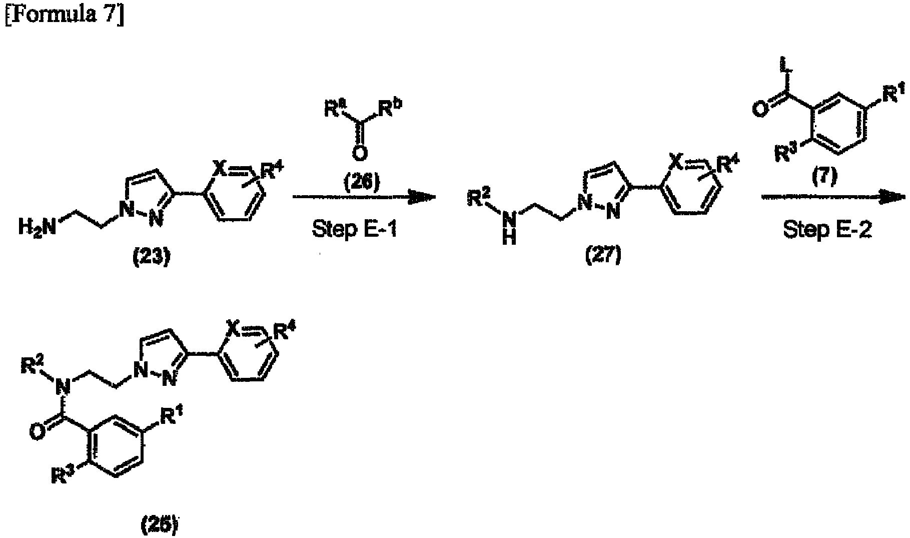

wherein X, R¹, R², R³, R⁴, and L are as defined above; and R^{a} and R^{b} are the same or different and each represent a hydrogen atom or an alkyl group.
Step E-1: A compound represented by formula (27) can be obtained by the reaction between an amine derivative represented by formula (23) and a compound represented by formula (26) under the same conditions of reductive amination reaction as in Step C-1.
Step E-2: A compound represented by formula (25) can be obtained by the reaction between the amine derivative represented by formula (27) and a compound represented by formula (7) according to the same reaction conditions as in Step A-4.

### Scheme F

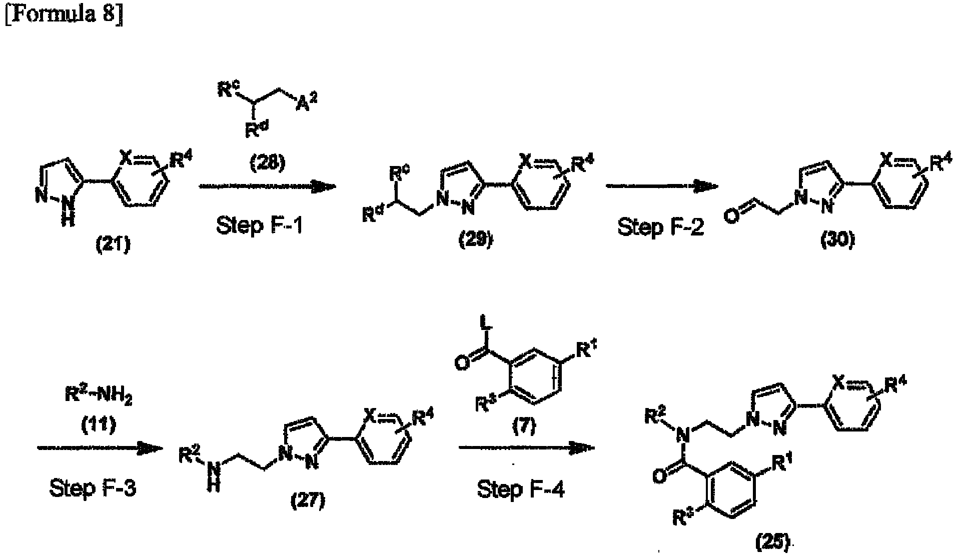

wherein X, R¹, R², R³, R⁴, A², and L are as defined above; and R^{c} and R^{d} each represent an alkoxy group.
Step F-1: A compound represented by formula (29) can be obtained by the reaction between a pyrazole derivative represented by formula (21) and a compound represented by formula (28) under the same reaction conditions as in Step A-2.
Step F-2: A compound represented by formula (30) can be obtained by the reaction between the compound represented by formula (29) and an acid such as hydrochloric acid, trifluoroacetic acid, or p-toluenesulfonic acid. Examples of the reaction solvent include solvents such as methanol, ethanol, tetrahydrofuran, 1,4-dioxane, water, ethyl acetate, chloroform, and acetone, and mixed solvents thereof. The reaction can be performed under a temperature condition of around -80°C to around the boiling point of the solvent.

Step F-3: A compound represented by formula (27) can be obtained by the reaction between the compound represented by formula (30) and an amine derivative represented by formula (11) under the same conditions of reductive amination reaction as in Step C-1.
Step F-4: A compound represented by formula (25) can be obtained by the reaction between the amine derivative represented by formula (27) and a compound represented by formula (7) according to the same reaction conditions as in Step A-4.

### Scheme G

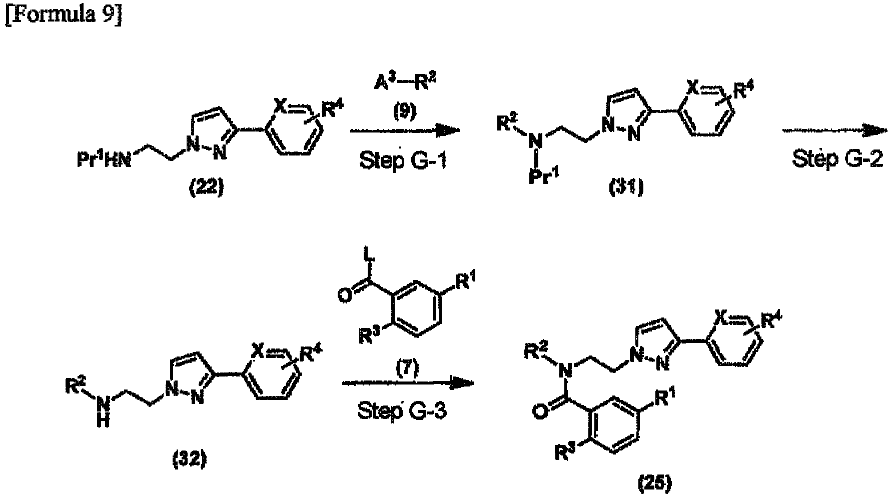

wherein X, R¹, R², R³, R⁴, A³, Pr¹, and L are as defined above.
Step G-1: A compound represented by formula (31) can be obtained by the reaction between a compound represented by formula (22) and a compound represented by formula (9) according to the same reaction conditions as in Step A-5.
Step G-2: A compound represented by formula (32) can be obtained by reaction from the compound represented by formula (31) according to the same reaction conditions as in Step A-3. Step G-3: A. compound represented by formula (25) can be obtained by the reaction between the amine derivative represented by formula (32) and a compound represented by formula (7) according to the same reaction conditions as in Step A-4.

### Scheme H

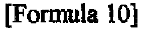

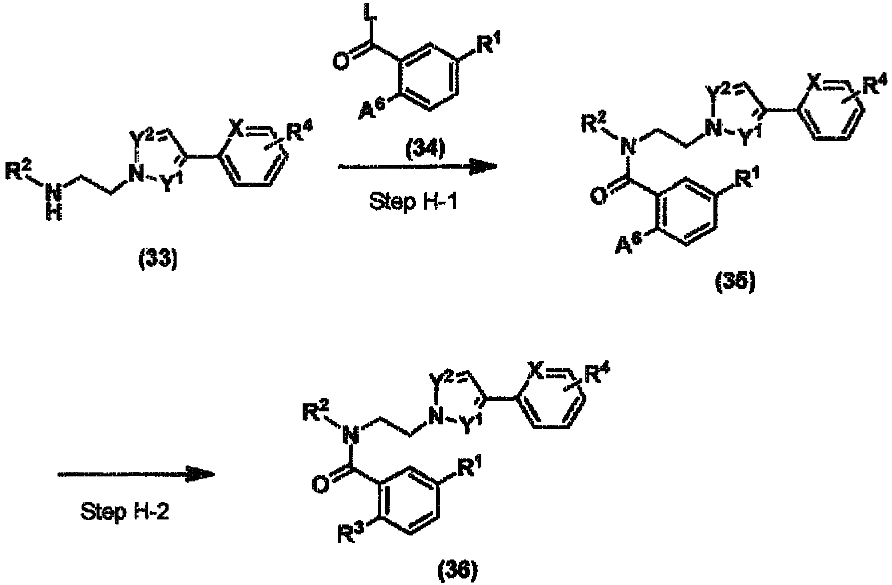

wherein X, Y¹, Y², R¹, R², R³, R⁴, and L are as defined above; and A⁶ represents a halogen atom. Step H-1: A compound represented by formula (35) can be obtained by the reaction between an amine derivative represented by formula (33) and a compound represented by formula (34) according to the same reaction conditions as in Step A-4.
Step H-2: A compound represented by formula (36) can be obtained by the reaction between the compound represented by formula (35) and a boronic acid derivative under the same conditions of Suzuki-Miyaura coupling reaction as in Step A-1. Alternatively, the compound represented by formula (36) may be obtained by reaction using an organic tin compound under conditions of Stille coupling reaction. The comprehensive overview of the Stille coupling reaction may be found in, for example, Angew. Chem. Int. Ed., 43, 4704, (2004).

### Scheme I

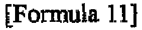

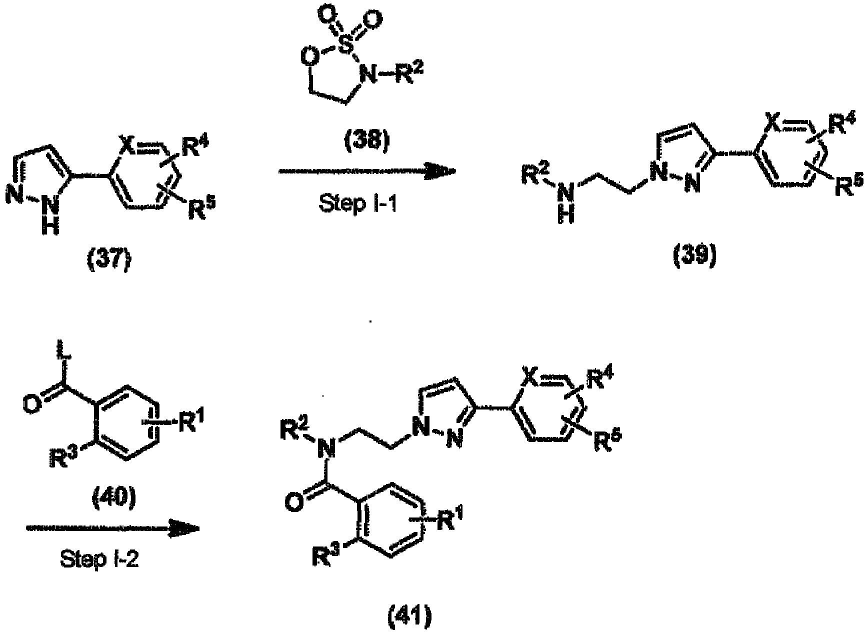

wherein X, R¹, R², R³, R⁴, and R⁵ are as defined above.
Step I-1: A compound represented by formula (39) can be obtained by the reaction between a compound represented by formula (37) and a compound represented by formula (38). The reaction in Step I-1 proceeds under a temperature condition of around -80°C to around the boiling point of a solvent in the presence of an inorganic base such as sodium hydroxide, potassium hydroxide, sodium carbonate, potassium carbonate, cesium carbonate, or sodium hydride or an organic base such as lower alkoxide of an alkali metal or an alkaline earth metal (e.g., sodium ethoxide and potassium tert-butoxide), triethylamine, or diisopropylethylamine in a solvent such as N,N-dimethylformamide, dimethyl sulfoxide, acetonitrile, tetrahydrofuran, or chloroform or in a mixed solvent thereof.
Step I-2: A compound represented by formula (41) can be obtained by the reaction between the amine derivative represented by formula (39) and a compound represented by formula (40) according to the same reaction conditions as in Step A-4.

### [Examples]

Hereinafter, the present invention will be described in more detail with reference to Reference Examples, Examples, and Test Example. However, these examples are not intended to limit the present invention. In addition, modifications or changes may be made therein without departing from the scope of the present invention.
In Reference Examples and Examples below, the microwave reactor used is Initiator from Biotage Japan Ltd.
In Reference Examples and Examples below, SNAPCartridge KP-Sil from Biotage Japan Ltd.. was used as "KP-Sil" for purification using column chromatography; SNAPCartridge HP-Sil from Biotage Japan Ltd. was used as "HP-Sil"; and SNAPCartridge KP-NH from Biotage Japan Ltd. was used as "KP-NH".

In Reference Examples and Examples below, purification by preparative high-performance liquid chromatography (HPLC) was performed under conditions shown below. However, in the case of a compound having a basic functional group, neutralization operation or the like may be performed in order to obtain a free form when trifluoroacetic acid is used in this operation.
Instrument: preparative HPLC system from Gilson, Inc.
Column: Capcell Pak C18 MG II 5 µm, 20 x 150 mm from Shiseido Co., Ltd.
Solvent: solution A; water containing 0.1% trifluoroacetic acid, solution B; acetonitrile containing 0.1% trifluoroacetic acid
Gradient: 0 min. (solution A/solution B = 90/10), 22 min. (solution A/solution B = 20/80), 25 min. (solution A/solution B = 10/90)
Flow rate: 20 mL/min, Detection method: UV 254 nm

In Reference Examples and Examples below, high-performance liquid chromatography mass spectra (LCMS) were measured under the following conditions:
Condition 1
   Measurement instrument: Platform LC from MicroMass Ltd. and Agilent 1100 from Agilent Technologies Inc.
   Column: SunFire C18 2.5 µm, 4.6 x 50 mm from Waters Corp.
   Solvent: solution A; water containing 0.1% trifluoroacetic acid, solution B; acetonitrile containing 0.1% trifluoroacetic acid
   Gradient: 0 min. (solution A/solution B = 90/10), 0.5 min. (solution A/solution B = 90/10), 5.5 min. (solution A/solution B = 20/80), 6.0 min. (solution A/solution B = 1/99), 6.3 min. (solution A/solution B = 1/99)
   Flow rate: 1 mL/min, Detection method: 254 nm
   Ionization method: electron spray ionization (ESI)
Condition 2
   Measurement instrument: Agilent 2900 from Agilent Technologies Inc. and Agilent 6150 from Agilent Technologies Inc.
   Column: Acquity CSH C18 1.7 um, 2.1 x 50 mm from Waters Corp.
   Solvent: solution A; water containing 0.1% formic acid, solution B; acetonitrile containing 0.1% formic acid
   Gradient: 0 min. (solution A/solution B = 80/20), 1.2-1.4 min. (solution A/solution B = 1/99) Flow rate: 0.8 mL/min, Detection method: 254 nm
   Ionization method: electron spray ionization (ESI)

In Reference Examples and Examples below, mass spectra (MS) were measured under the following conditions:
MS measurement instrument: LCMS-2010EV from Shimadzu Corp. or Platform LC from MicroMass Ltd.
In Reference Examples and Examples below, each compound was designated using ACD/Name (ACD/Labs 12.01, Advanced Chemistry Development Inc.).

In Reference Examples and Examples, the following terms and reagents are indicated:
Na₂SO₄ (anhydrous sodium sulfate), Na₂CO₃ (sodium carbonate), Cs₂CO₃ (cesium carbonate), NaHCO₃ (sodium bicarbonate), NaOH (sodium hydroxide), MeOH (methanol), EtOH (ethanol), Et₂O (diethyl ether), THF (tetrahydrofuran), DMF (N,N-dimethylformamide), MeCN (acetonitrile), EtOAc (ethyl acetate), CHCl₃ (chloroform), HOBt·H₂O (1-hydroxybenzotriazole monohydrate), EDC·HCl [1-(3-dimethylaminopropyl)-3-ethylcarbodiimide monohydrochloride], HATU [O-(7-azabenzotriazol-1-yl)-N,N,N',N'-tetramethyluronium hexafluorophosphate], Pd(PPh₃)₄ [tetrakis(triphenylphosphine)palladium(0)], brine (saturated saline), Boc (tert-butoxycarbonyl), THP (tetrahydropyranyl), DIPEA (N,N-diisopropylethylamine), TEA. (triethylamine), MeI (methyl iodide), EtI (ethyl iodide), TBS (tert-butyldimethylsilyl), TBAF (tetrabutylammonium fluoride), MsCl (methanesulfonyl chloride), NaBH₄ (sodium borohydride), NaH (sodium hydride), and HCl (hydrogen chloride).

### Reference Example 1 5-Fluoro-2-[1-(tetrahydro-2H-pyran-2-yl)-1H-pyrazol-5-yl]pyridine

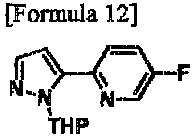

Pd(PPh₃)₄ (0.42 g, 0.36 mmol) and a 2 mol/L aqueous Na₂CO₃ solution (4 mL) were added to an EtOH (10 mL)/toluene (10 mL) mixed solution of 1-(tetrahydro-2H-pyran-2-yl)-5-(4,4,5,5-tetramethyl-1,3,2-dioxaborolan-2-yl)-1H-pyrazole (1.0 g, 3.6 mmol) and 2-bromo-5-fluoropyridine (0.82 g, 4.7 mmol), and the mixture was heated to reflux and stirred for 2 hours. After standing to cool at room temperature, water was added to the reaction mixture, followed by extraction with EtOAc. The extracted organic layer was washed with brine and dried over Na₂SO₄. Then, the desiccant was filtered off, and the solvent was distilled off under reduced pressure. The obtained residue was purified by column chromatography (KP-NH 28 g, hexane/EtOAc = 80/20 → 20/80) to obtain the title compound (0.84 g) (colorless oil).
MS (ESI pos.) m/z: 248 [M+H]+

### Reference Example 2 5-Fluoro-2-(1H-pyrazol-5-yl)pyridine hydrochloride

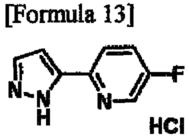

A 4 mol/L HCl-EtOAc solution (5 mL) was added to a solution of the compound (0. 84 g, 3.4 mmol) obtained in Reference Example 1 in MeOH (10 mL), and the mixture was stirred at room temperature for 16 hours and at 60°C for 3 hours. After standing to cool at room temperature, the solvent in the reaction mixture was distilled off under reduced pressure. The obtained residue was stirred for 1 hour in Et₂O. Then, the deposited solid was collected by filtration and dried by heating under reduced pressure to obtain the title compound (0.62 g) (colorless powder).
MS (ESI pos.) m/z: 1.64 [M+H]+, 186 [M+Na]+

### Reference Example 3 Tert-butyl {2-[3-(5-fluoropyridin-2-yl)-1H-pyrazol-1-yl]ethyl}carbamate

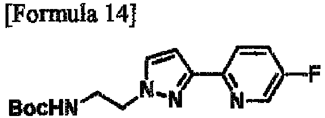

Tert-butyl N-(2-bromoethyl)carbamate (1.9 g, 8.5 mmol) and Cs₂CO₃ (6.4 g, 19.5 mmol) were added to a solution of the compound (1.3 g, 6.5 mmol) obtained in Reference Example 2 in DMF (50 mL), and the mixture was stirred at 80°C for 3 hours. After standing to cool at room temperature, water was added to the reaction mixture, followed by extraction with EtOAc. The extracted organic layer was washed with brine and dried over Na₂SO₄. Then, the desiccant was filtered off, and the solvent was distilled off under reduced pressure. The obtained residue was purified by column chromatography (KP-NH 110 g, hexane/EtOAc = 100/0 → 80/20) to obtain the title compound (1.3 g) (colorless oil).
MS (ESI pos.) m/z: 307 [M+H]+, 329 [M+Na]+

### Reference Example 4 2-[3-(5-Fluoropyridin-2-yl)-1H-pyrazol-1-yl]ethanamine dihydrochloride

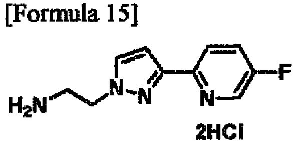

A 4 mol/L HCl-EtOAc solution (5 mL) was added to a solution of the compound (1.3 g, 4.2 mmol) obtained in Reference Example 3 in CHCl₃ (20 mL), and the mixture was stirred at room temperature for 24 hours. The solvent in the reaction mixture was distilled off under reduced pressure, and the obtained residue was stirred for 1 hour in Et₂O. Then, the deposited solid was collected by filtration and dried by heating under reduced pressure to obtain the title compound (0.86 g) (colorless powder).
MS (ESI pos.) m/z: 207 [M+H]+

### Reference Example 5 5-Fluoro-2-(1H-pyrazol-4-yl)pyridine

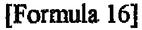

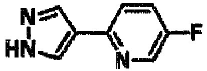

A 2 mol/L aqueous Na₂CO₃ solution (8.5 mL, 17.1 mmol) and Pd(PPh₃)₄ (0.20 g, 0.17 mmol) were added to a solution of tert-butyl 4-(4,4,5,5-tetramethyl-1,3,2-dioxaborolan-2-yl)-1H-pyrazole-1-carboxylate (1.8 g, 6.3 mmol) and 2-bromo-5-fluoropyridine (1.0 g, 5.7 mmol) in 1,4-dioxane (11 mL), and the mixture was stirred in an oil bath with a temperature of 90°C for 4 hours. Then, the mixture was stirred at room temperature for 2 days. Brine was added to the reaction mixture, followed by extraction with EtOAc. The organic layer was dried over Na₃SO₄, and the desiccant was filtered off. Then, the solvent was distilled off under reduced pressure. EtOAc was added to the obtained residue, and the deposited solid was collected by filtration to obtain the title compound (0.66 g) (colorless solid).
MS (ESI pos.) m/z: 164 [M+H]+

### Reference Example 6 Tert-butyl {2-[4-(5-fluoropyridin-2-yl)-1H-pyrazol-1-yl]ethyl}carbamate

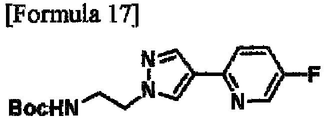

A mixture of the compound (0.20 g, 1.2 mmol) obtained in Reference Example 5, tert-butyl (2-bromoethyl)carbamate (0.30 g, 1.4 mmol), Cs₂CO₃ (0.80 g, 2.5 mmol), and MeCN (2.5 mL) was stirred in an oil bath with a temperature of 80°C for 1 hour. Then, tert-butyl (2-bromoethyl)carbamate (0.030 g, 0.13 mmol) was added thereto, and the mixture was stirred at the same temperature as above for 2 hours. After standing to cool to room temperature, water was added thereto, followed by extraction with EtOAc. The organic layer was dried over Na₂SO₄, and the desiccant was filtered off. Then, the solvent was distilled off under reduced pressure to obtain the title compound (0.34 g) (colorless solid).
MS (ESI pos.) m/z: 307 [M+H]+, 329 [M+Na]+

### Reference Example 7 2-[4-(5-Fluoropyridin-2-yl)-1H-pyrazol-1-yl]ethanamine dihydrochloride

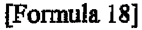

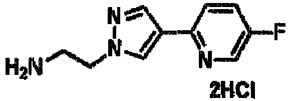

A 4 mol/L HCl-EtOAc solution (5.5 mL) was added to a solution of the compound (0.34 g, 1.1 mmol) obtained in Reference Example 6 in EtOAc (1 mL) at room temperature. EtOAc (4 mL) was added to the reaction mixture, and the mixture was stirred at the same temperature as above for 1 hour. Then, a 4 mol/L HCl-EtOAc solution (2.5 mL) was added thereto, and the mixture was stirred at the same temperature as above for 2 hours. The deposited solid was collected by filtration and washed with EtOAc to obtain the title compound (0.29 g) (colorless solid).
MS (ESI pos.) m/z: 207 [M+H]+

### Reference Example 8 N-ethyl-5-methyl-2-(2H-1,2,3-triazol-2-yl)benzamide

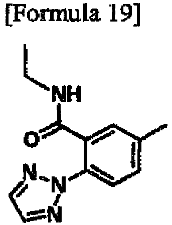

HOBt·H₂O (4.05 g, 26.6 mmol) and EDC·HCl (5.1 g, 26.6 mmol) were added to a solution of 5-methyl-2-(2H-1,2,3-triazol-2-yl)benzoic acid (3.0 g, 14.8 mmol) and ethylamine (2 mol/L solution in THF, 8.9 mL, 17.8 mmol) in DMF (54 mL) at room temperature, and the mixture was stirred overnight. An aqueous NaHCO₃ solution was added to the reaction mixture, followed by extraction with EtOAc. The organic layer was washed with water and brine, dried over Na₂SO₄, and the desiccant was filtered off. Then, the solvent was distilled off under reduced pressure. The obtained residue was purified by column chromatography (KP-Sil 100 g, hexane/EtOAc = 85/15 → 0/100). The obtained solid was washed with a hexane/EtOAc mixed solvent (9/1) with stirring and then collected by filtration to obtain the title compound (2.5 g) (colorless solid).
MS (ESI pos.) m/z: 231 [M+H]+, 253 [M+Na]+

### Reference Example 9 N-(2-{[tert-butyl(dimethyl)silyl]oxy}ethyl)-N-ethyl-5-methyl-2-(2H-1,2,3-triazol-2-yl)benzamide

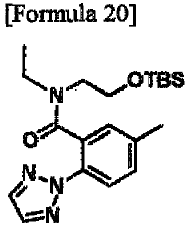

60% NaH (0.47 g, 10.9 mmol) was added to a solution of the compound (2.0 g, 8.7 mmol) obtained in Reference Example 8 in DMF (87 mL) at room temperature, and the mixture was stirred for 30 minutes. A solution of (2-bromoethoxy)-tert-butyldimethylsilane (2.60 g, 10.9 mmol) in DMF (1 mL) was added to the reaction mixture at room temperature, and the mixture was stirred at room temperature for 1 hour and then stirred in an oil bath with a temperature of 50°C for 4 hours. Then, the mixture was stirred at room temperature for 2 days. Water was added to the reaction mixture, followed by extraction with EtOAc. The organic layer was washed with water and brine, dried over Na₂SO₄, and the desiccant was filtered off. Then, the solvent was distilled off under reduced pressure. The obtained residue was purified by column chromatography (KP-Sil 100 g, hexane/EtOAc = 85/15 → 0/100) to obtain the title compound (3.6 g) (brown oil).
MS (ESI pos.) m/z: 389 [M+H]+

### Reference Example N-ethyl-N-(2-hydroxyethyl)-5-methyl-2-(2H-1,2,3-triazol-2-yl)benzamide

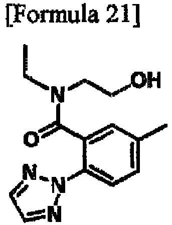

1 mol/L TBAF (THF solution, 2.3 mL, 2.3 mmol) was added to a solution of the compound (0.76 g, 2.0 mmol) obtained in Reference Example 9 in. THF (10 mL) under cooling in ice water, and the mixture was heated to room temperature and stirred for 1 hour. After cooling in ice water again, an aqueous NaHCO₃ solution was added thereto, and THF was distilled off under reduced pressure. EtOAc was added to the residue for extraction. The organic layer was washed with brine and then dried over Na₂SO₄. The desiccant was filtered off, and the solvent was then distilled off under reduced pressure. The obtained residue was purified by column chromatography (KP-Sil 25 g, hexane/EtOAc = 70/30 → 0/100) to obtain the title compound (0.53 g) (colorless solid).
MS (ESI pos.) m/z: 275 [M+H]+, 297 [M+Na]+

### Reference Example 11 N-(2-{[tert-butyl(dimethyl)silyl]oxy}ethyl)cyclopropanamine

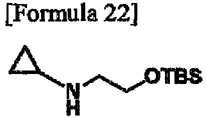

{[Tert-butyl(dimethyl)silyl]oxy}-acetaldehyde (0.30 mL, 1.6 mmol) was added to a solution of cyclopropylamine (0.090 g, 1.6 mmol) in MeOH (3.2 mL) at room temperature, and the mixture was stirred for 1 hour. NaBH₄ (0.071 g, 1.9 mmol) was added thereto at the same temperature as above, and the mixture was stirred for 3 hours. Water was added to the reaction mixture, and MeOH was distilled off under reduced pressure. Then, brine was added to the residue, followed by extraction with EtOAc. The organic layer was dried over Na₂SO₄, and the desiccant was filtered off. Then, the solvent was distilled off under reduced pressure to obtain the title compound (0.32 g) (oil). The compound was used in the next reaction without being further purified.
MS (ESI pos.) m/z: 216 [M+H]+

### Reference Example 12 N-(2-{[tert-butyl(dimethyl)silyl]oxy}ethyl)-N-cyclopropyl-5-methyl-2-(2H-1,2,3-triazol-2-yl)benzamide

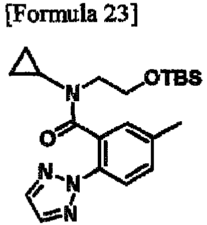

DIPEA (0.78 mL, 4.5 mmol) and HATU (0.68 g, 1.8 mmol) were added to a solution of the compound (0.32 g, 1.5 mmol) obtained in Reference Example 11 and 5-methyl-2-(2H-1,2,3-triazol-2-yl)benzoic acid (0.31 g, 1.5 mmol) in DMF (4.5 mL) at room temperature, and the mixture was stirred for 15 hours. Water was added to the reaction mixture, followed by extraction with EtOAc. The organic layer was washed with water and brine, and the solvent was distilled off under reduced pressure. The obtained residue was purified by column chromatography (KP-NH 28 g, hexane/EtOAc = 80/20 → 0/100) and further purified (KP-Sil 25 g, hexane/EtOAc = 95/5 → 0/100) to obtain the title compound (0.33 g) (colorless amorphous). MS (ESI pos.) m/z: 401 [M+H]+, 423 [M+Na]+

### Reference Example 13 N-cyclopropyl-N-(2-hydroxyethyl)-5-methyl-2-(2H-1,2,3-triazol-2-yl)benzamide

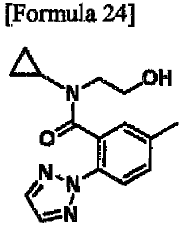

The title compound (0.21 g) was obtained (colorless amorphous) by the same approach as in Reference Example 10 using the compound (0.33 g, 0.82 mmol) obtained in Reference Example 12 as a starting material.
MS (ESI pos.) m/z: 287 [M+H]+, 309 [M+Na]+

### Reference Example 14 Tert-butyl ethyl{2-[3-(5-fluoropyridin-2-yl)-1H-pyrazol-1-yl]ethyl}carbonate

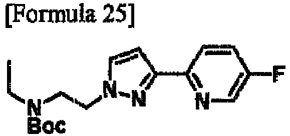

60% NaH (0.056 g, 1.4 mmol) was added to a solution of the compound (0.35 g, 1.1 mmol) obtained in Reference Example 3 in DMF (5 mL), and the mixture was stirred at room temperature for 30 minutes. EtI (0.11 mL, 1.4 mmol) was added thereto, and the mixture was stirred at room temperature for 1 hour. A saturated aqueous solution of ammonium chloride was added to the reaction mixture, followed by extraction with EtOAc. The extracted organic layer was washed with brine and dried over Na₂SO₄. Then, the desiccant was filtered off, and the solvent was distilled off under reduced pressure to obtain the title compound (0.40 g) (pale yellow oil).
MS (ESI pos.) m/z: 335 [M+H]+

### Reference Example 15-1 N-ethyl-2-[3-(5-fluoropyridin-2-yl)-1H-pyrazol-1-yl]ethanamine dihydrochloride

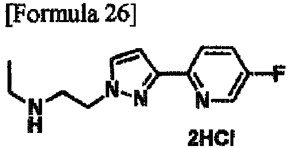

A 4 mol/L HCl-EtOAc solution (2 mL) was added to a solution of the compound (0.37 g, 1.1 mmol) obtained in Reference Example 14 in CHCl₃ (5 mL), and the mixture was stirred at room temperature for 24 hours. The solvent in the reaction mixture was distilled off under reduced pressure, and the obtained residue was stirred for 1 hour in Et₂O. Then, the deposited solid was collected by filtration and dried by heating under reduced pressure to obtain the title compound (0.23 g) (colorless powder).
MS (ESI pos.) m/z: 234 [M+H]+

### Reference Example 15-2 N-ethyl-2-[3-(5-fluoropyridin-2-yl)-1H-pyrazol-1-yl]ethanamine dihydrochloride

Alternatively, the title compound may be obtained as follows:
A solution of 3-ethyl-1,2,3-oxathiazolidine-2,2-dioxide (2.27 g, 15.0 mmol) in MeCN (3.2 mL) was added dropwise to a mixture of the compound (2.00 g, 10.0 mmol) obtained in Reference Example 2, NaOH (1.00 g, 25 mmol), and MeCN (22 mL) at 67 to 71°C, and the mixture was stirred at the same temperature as above for 1 hour. After ice cooling, 25% H₂SO₄ (12 mL) was added thereto at 18°C. The reaction mixture was heated and stirred at 68°C for 4 hours. After ice cooling, a 10 mol/L aqueous NaOH solution (8.5 mL) was added thereto, and the mixture was then separated into aqueous and organic layers. The solvent in the organic layer was distilled off under reduced pressure. The residue was stirred in EtOAc (16 mL), and a 4 mol/L HCl-EtOAc solution (5.3 mL) was added thereto under ice cooling. The mixture was stirred at room temperature for 1 hour, and the deposited solid was then collected by filtration.. The obtained solid was dried by heating under reduced pressure to obtain the title compound (2.49 g) (pale yellow crystal).

### Reference Example 16 N-{2-[3-(5-fluoropyridin-2-yl)-1H-pyrazol-1-yl]ethy}propan-2-amine

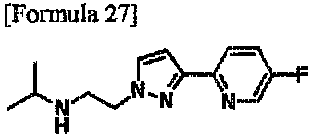

The compound (0.32 g, 1.1 mmol) obtained in Reference Example 4 was dissolved in water, and a 2 mol/L aqueous NaOH solution was then added to the solution, followed by extraction with CHCl₃. The extracted organic layer was washed with brine and dried over Na₂SO₄. Then, the desiccant was filtered off, and the solvent was distilled off under reduced pressure. Acetone (0.25 mL, 3.4 mmol) was added to a solution of the obtained residue in EtOH (5 mL), and the mixture was stirred at 60°C for 30 minutes. After standing to cool at room temperature, NaBH₄ (0.13 g, 3.4 mmol) was added thereto, and the mixture was stirred at 60°C for 2 hours. After standing to cool at room temperature, water was added to the reaction mixture, followed by extraction with EtOAc. The organic layer was washed with brine and dried over Na₂SO₄. Then, the desiccant was filtered off, and the solvent was distilled off under reduced pressure to obtain the title compound (0.11 g) (pale yellow oil).
MS ((ESI pos.) m/z: 249 [M+H]+

### Reference Example 17 N-{2-[3-(5-fluoropyridin-2-yl)-1H-pyrazol-1-yl]ethyl}cyclopropanamine

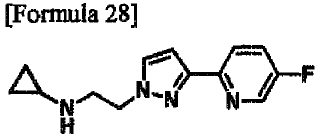

Bromoacetaldehyde diethyl acetal (1.5 mL, 9.8 mmol) and Cs₂CO₃ (7.3 g, 22.5 mmol) were added to a solution of the compound (1.5 g, 7.5 mmol) obtained in Reference Example 2 in DMF (50 mL), and the mixture was stirred at 80°C for 4 hours. After standing to cool at room temperature, water was added to the reaction mixture, followed by extraction with EtOAc. The extracted organic layer was washed with brine and dried over Na₂SO₄. Then, the desiccant was filtered off, and the solvent was distilled off under reduced pressure. The obtained residue was purified by column chromatography (KP-Sil 100 g, hexane/EtOAc = 100/0 → 80/20) to obtain 2-[1-(2,2-diethoxyethyl)-1H-pyrazol-3-yl]-5-fluoropyridine (1.3 g) in a colorless oil form. Trifluoroacetic acid (2.0 mL, 27.9 mmol) was added dropwise to a solution of the obtained 2-[1-(2,2-diethoxyethyl)-1H-pyrazol-3-yl]-5-fluoropyridine (1.3 g, 4.7 mmol) in CHCl₃ (20 mL) under ice cooling. After the completion of dropwise addition, the mixture was heated to room temperature and stirred for 20 hours. A saturated aqueous solution of NaHCO₃ was added to the reaction mixture, followed by extraction with CHCl₃. The organic layer was washed with brine and dried over Na₂SO₄. Then, the desiccant was filtered off, and the solvent was distilled off under reduced pressure to obtain [3-(5-fluoropyridin-2-yl)-1H-pyrazol-1-yl]acetaldehyde (0.6 g) (pale yellow oil). Cyclopropylamine (0.092 mg, 1.6 mmol) was added to a solution of the obtained [3-(5-fluoropyridin-2-yl)-1H-pyrazol-1-yl]acetaldehyde (0.3 g, 1.5 mmol) in CHCl₃ (10 mL), and the mixture was stirred at room temperature for 30 minutes. Sodium triacetoxyborohydride (0.95 g, 4.5 mmol) was added thereto, and the mixture was stirred at room temperature for 24 hours. A saturated aqueous solution of NaHCO₃ was added to the reaction solution, followed by extraction with CHCl₃. The organic layer was washed with brine and dried over Na₂SO₄. Then, the desiccant was filtered off, and the solvent was distilled off under reduced pressure. The obtained residue was purified by column chromatography (KP-NH 28 g, hexane/EtOAc = 80/20 → 20/80) to obtain the title compound (0.20 g) (colorless oil). MS (ESI pos.) m/z: 247 [M+H]+

### Reference Example 18 Tert-butyl ethyl{2-[4-(5-fluoropyridin-2-yl)-1H-pyrazol-1-yl]ethyl} carbamate

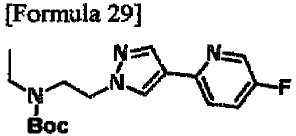

The title compound (1.4 g) was obtained (colorless amorphous) in the same way as in Reference Example 14 using the compound (1.2 g, 3.9 mmol) obtained in Reference Example 6.
MS (ESI pos.) m/z: 335 [M+H]+

### Reference Example 19 N-ethyl-2-[4-(5-fluoropyridin-2-yl)-1H-pyrazol-1-yl]ethanamine

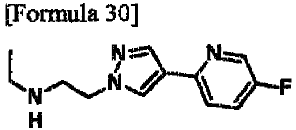

A 4 mol/L HCl-EtOAc solution (20.3 mL) was added to a solution of the compound (1.4 g, 4.1 mmol) obtained in Reference Example 18 in EtOAc (5 mL) at room temperature. EtOAc (5 mL) was added to the reaction mixture, and the mixture was stirred at the same temperature as above for 4 hours. The reaction mixture was rendered basic by the addition of a 2 mol/L aqueous NaOH solution, followed by extraction with EtOAc. The organic layer was washed with brine and dried over Na₃SO₄. Then, the desiccant was filtered off, and the solvent was distilled off under reduced pressure. Et₂O was added to the obtained residue, and the deposited solid was collected by filtration to obtain the title compound (0.81 g) (colorless solid).
MS (ESI pos.) m/z: 235 [M+H]+

### Reference Example 20 N-{2-[3-(5-fluoropyridin-2-yl)-1H-pyrazol-1-yl]ethyl}-2-iodo-5-methylbenzamide

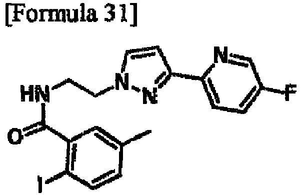

The title compound (1.5 g) was obtained (colorless solid) by the same approach as in Reference Example 8 using the compound (1.0 g, 3.6 mmol) obtained in Reference Example 4 and 2-iodo-5-methylbenzoic acid (0.97 g, 3.8 mmol) as starting materials.
MS (ESI pos.) m/z: 451 [M+H]+

### Reference Example 21 N-ethyl-N-{2-[3-(5-fluoropyridin-2-yl)-1H-pyrazol-1-yl]ethyl}-2-iodo-5-methylbenzamide

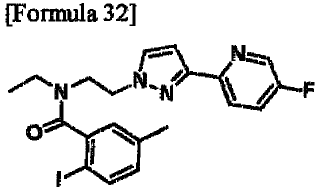

The title compound (0.67 g) was obtained (colorless amorphous) by the same approach as in Reference Example 14 using the compound (0.70 g, 1.8 mmol) obtained in Reference Example 20 as a starting material.
MS (ESI pos.) m/z: 479 [M+H]+

### Reference Example 22 Tert-butyl {2-[4-(4-fluorophenyl)-1H-pyrazol-1-yl]ethyl}carbamate

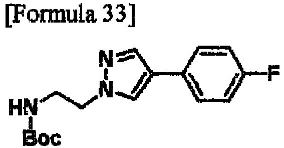

The title compound (0.22 g, 0.71 mmol) was obtained (colorless solid) by the same approach as in Reference Example 6 using 4-(4-fluorophenyl)-1H-pyrazole (0.12 g, 0.72 mmol) as a starting material.
MS (ESI pos.) m/z: 306 [M+H]+, 328 [M+Na]+

### Reference Example 23 2-[4-(4-Fluorophenyl)-1H-pyrazol-1-yl]ethanamine hydrochloride

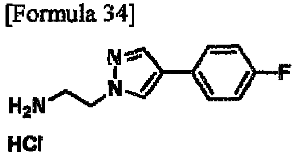

The title compound (0.17 g, 0.71 mmol) was obtained (colorless solid) by the same approach as in Reference Example 7 using the compound (0.22 g, 0.71 mmol) obtained in Reference Example 22 as a starting material.
MS (ESI pos.) m/z: 206 [M+H]+

### Reference Example 24 N-(2-{[tert-butyl(dimethyl)sityl]oxy}ethyl)-5-methyl-N-(propan-2-yl)-2-(2H-1,2,3-triazol-2-yl)benzamide

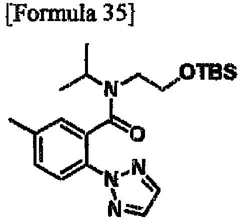

The title compound (0.35 g, 0.87 mmol) was obtained (colorless solid) by the same approach as in Reference Example 12 using N-(2-{[tert-butyl(dimethyl)silyl]oxyl}ethyl)propan-2-amine (0.33 g, 1.5 mmol) as a starting material. MS (ESI pos.) m/z: 403 [M+H]+

### Reference Example 25 N-(2-hydroxyethyl)-5-methyl-N-(propan-2-yl)-2-(2H-1,2,3-triazol-2-yl)benzamide

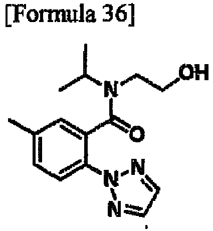

The title compound (0.15 g, 0.52 mmol) was obtained (colorless solid) by the same approach as in Reference Example 13 using the compound (0.35 g, 0.87 mmol) obtained in Reference Example 24 as a starting material.
MS (ESI pos.) m/z: 289 [M+H]+

### Reference Example 26 N-(cyclopropylmethyl)-2-[3-(4-fluorophenyl)-1H-pyrazol-1-yl]ethanamine

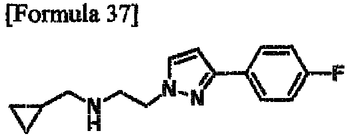

The title compound (0.41 g) was obtained (colorless oil) by the same approach as in Reference Example 16 using 2-[3-(4-fluorophenyl)-1H-pyrazol-1-yl]ethanamine hydrochloride (0.43 g, 2.1 mmol) and cyclopropanecarbaldehyde (0.17 mL, 2.3 mmol) as starting materials.
MS (ESI pos.) m/z: 260 [M+H]+

### Reference Example 27 N-(cyclopropylmethyl)-2-(3-phenyl-1H-pyrazol-1-yl)ethanamine

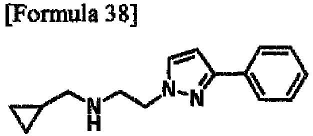

The title compound (0.31 g) was obtained (colorless oil) by the same approach as in Reference Example 16 using 2-(3-phenyl-1H-pyrazol-1-yl)ethanamine (0.37 g, 2.0 mmol) and cyclopropanecarbaldehyde (0.16 mL, 2.2 mmol) as starting materials.
MS (ESI pos.) m/z: 242 [M+H]+

### Reference Example 28 N-(oxetan-3-yl)-2-[4-(5-fluoropyridin-2-yl)-1H-pyrazol-1-yl]ethanamine

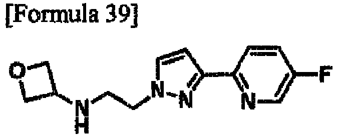

Oxetan-3-amine hydrochloride (0.092 mg, 1.6 mmol) was added to a solution of [3-(5-fluoropyridin-2-yl)-1H-pyrazol-1-yl]acetaldehyde (0.30 g, 1.5 mmol) obtained in Reference Example 17 in CHCl₃ (10 mL), and the mixture was stirred at room temperature for 30 minutes. Sodium triacetoxyborohydride (0.95 g, 4.5 mmol) was added thereto, and the mixture was stirred at room temperature for 24 hours. A saturated aqueous solution of NaHCO₃ was added to the reaction solution, followed by extraction with CHCl₃. The extracted organic layer was washed with brine and dried over Na₂SO₄. Then, the desiccant was filtered off, and the solvent was distilled off under reduced pressure. The obtained residue was purified by column chromatography (KP-NH 28 g, hexane/EtOAc = 80/20 → 20/80) to obtain the title compound (0.20 g) (colorless oil).
MS (ESI pos.) m/z: 263 [M+H]+

### Reference Example 29 2-{[3-(6-Methylpyridin-2-yl)-1H-pyrazol-1-yl]ethyl}-1H-isoindole-1,3(2H)-dione

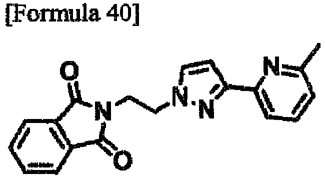

The title compound (0.13 g) was obtained (colorless oil) by the same approach as in Reference Example 6 using 2-methyl-6-(1H-pyrazol-3-yl)pyridine (0.46 g, 2.35 mmol) and N-(2-bromoethyl)phthalimide (0.72 g, 2.82 mmol) as starting materials.
MS (ESI pos.) m/z: 333 [M+H]+

### Reference Example 30 2-[3-(6-Methylpyridin-2-yl)-1H-pyrazol-1-yl]ethanamine

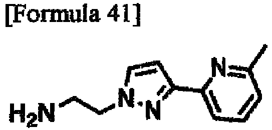

Hydrazine monohydrate (0.038 ml, 0.78 mmol) was added to a solution of the compound (0.13 g, 0.39 mmol) obtained in Reference Example 29 in ethanol (5 ml), and the mixture was heated to reflux and stirred for 5 hours. After standing to cool at room temperature, the deposited solid was filtered off, and the solvent in the filtrate was distilled off under reduced pressure to obtain the title compound (0.080 g) (colorless oil).
MS (ESI pos.) m/z: 203 [M+H]+

### Reference Example 31 5-Trifluoromethyl-2-[1-(tetrahydro-2H-pyran-2-yl)-1H-pyrazol-5-yl]pyridine

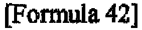

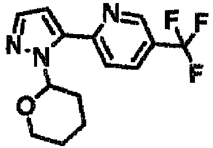

The title compound (0.66 g) was obtained by the same approach as in Reference Example 1 using 1-(tetrahydro-2H-pyran-2-yl)-5-(4,4,5,5-tetramethyl-1,3,2-dioxaborolan-2-yl)-1H-pyrazole (0.68 g, 2.43 mmol) and 2-bromo-5-trifluoromethylpyridine (0.50 g, 2.21 mmol) as starting materials.
MS (ESI pos.) m/z: 320 [M+Na]+

### Reference Example 32 5-Trifluoromethyl-2-(1H-pyrazol-5-yl)pyridine hydrochloride

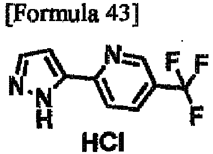

The title compound (0.38 g) was obtained by the same approach as in Reference Example 2 using the compound (0.66 g, 2.22 mmol) obtained in Reference Example 31 as a starting material.
MS (ESI pos.) m/z: 214 [M+H]+

### Reference Example 33 N-ethyl-2-[3-(5-trifluoromethylpyridin-2-yl)-1H-pyrazol-1-yl]ethanamine

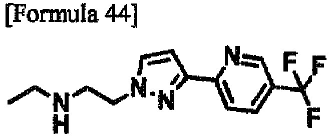

The title compound (0.030 g) was obtained by the same approach as in Reference Example 15-2 using the compound (0.15 g, 0.60 mmol) obtained in Reference Example 32 as a starting material.
MS (ESI pos.) m/z: 285 [M+H]+

### Reference Example 34 6-Trifluoromethyl-2-[1-(tetrahydro-2H-pyran-2-yl)-1H-pyrazol-5-yl]pyridine

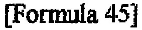

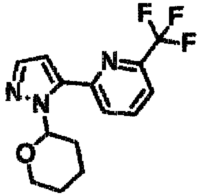

The title compound (0.62 g) was obtained by the same approach as in Reference Example 1 using 1-(tetrahydro-2H-pyran-2-yl)-5-(4,4,5,5-tetramethyl-1,3,2-dioxaborolan-2-yl)-1H-pyrazole (0.63 g, 2.29 mmol) and 2-bromo-6-trifluoromethylpyridine (0.47 g, 2.08 mmol) as starting materials.
MS (ESI pos.) m/z: 320 [M+Na]+

### Reference Example 35 6-Trifluoromethyl-2-(1H-pyrazol-5-yl)pyridine hydrochloride

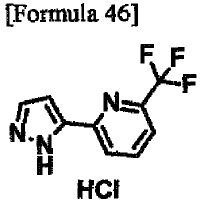

The title compound (0.50 g) was obtained by the same approach as in Reference Example 2 using the compound (0.62 g, 2.29 mmol) obtained in Reference Example 34 as a starting material.
MS (ESI pos.) m/z: 214 [M+H]+

### Reference Example 36 N-ethyl-2-[3-(6-trifluoromethylpyridin-2-yl)-1H-pyrazol-1-yl]ethanamine hydrochloride

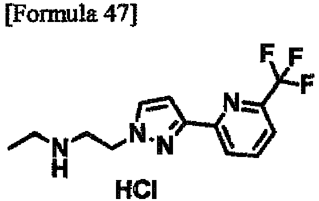

The title compound (0.12 g) was obtained by the same approach as in Reference Example 15-2 using the compound (0.50 g, 0.21 mmol) obtained in Reference Example 35 as a starting material.
MS (ESI pos.) m/z: 285 [M+H]+

### Reference Example 37 N-ethyl-2-[3-(3,4-difluorophenyl)-1H-pyrazol-1-yl]ethanamine hydrochloride

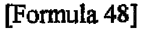

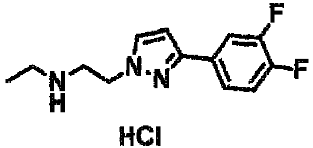

The title compound (0.11 g) was obtained by the same approach as in Reference Example 15-2 using 5-(3,4-difluorophenyl)-1H-pyrazole (0.50 g, 2.78 mmol) as a starting material.
MS (ESI pos.) m/z: 252 [M+H]+

### Reference Example 38 N-ethyl-2-[3-(4-fluorophenyl)-1H-pyrazol-1-yl]ethanamine hydrochloride

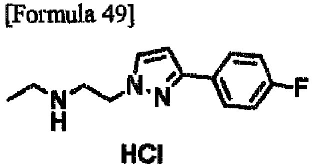

The title compound (0.13 g) was obtained by the same approach as in Preference Example 15-2 using 5-(4-fluorophenyl)-1H-pyrazole (0.50 g, 3.08 mmol) as a starting material.
MS (ESI pos.) m/z: 234 [M+H]+

### Reference Example 39 4-Trifluoromethyl-2-[1-(tetrahydro-2H-pyran-2-yl)-1H-pyrazol-5-yl]pyridine

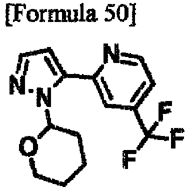

The title compound (1.31 g) was obtained by the same approach as in Reference Example 1 using 1-(tetrahydro-2H-pyran-2-yl)-5-(4,4,5,5-tetramethyl-1,3,2-dioxaborolan-2-yl)-1H-pyrazole (1.35 g, 4.87 mmol) and 2-bromo-4-trifluoromethylpyridine (1.0 g, 4.42 mmol) as starting materials.
MS (ESI pos.) m/z: 320 [M+Na]+

### Reference Example 40 4-Trifluoromethyl-2-(1H-pyrazol-5-yl)pyridine hydrochloride

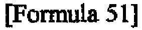

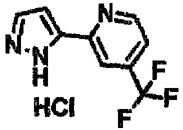

The title compound (0.80 g) was obtained by the same approach as in Reference Example 2 using the compound (1.31 g, 4.42 mmol) obtained in Reference Example 39 as a starting material.
MS (ESI pos.) m/z: 214 [M+H]+

### Reference Example 41 N-ethyl-2-[3-(4-trifluoromethylpyridin-2-yl)-1H-pyrazol-1-yl]ethanamine dihydrochloride

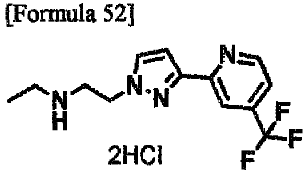

The title compound (0.73 g) was obtained by the same approach as in Reference Example 15-2 using the compound (0.60 g, 2.40 mmol) obtained in Reference Example 40 as a starting material.
MS (ESI pos.) m/z: 285 [M+H]+

### Example 1 N-{2-[4-(5-fluoropyridin-2-yl)-1H-pyrazol-1-yl]ethyl}-5-methyl-2-(2H-1,2,3-triazol-2-yl)benzamide

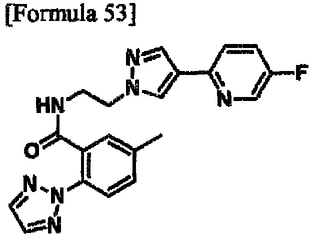

DIPEA (0.62 mL, 3.6 mmol) was added to a solution of the compound (0.20 g, 0.72 mmol) obtained in Reference Example 7 in DMF (2.2 mL) at room temperature 5-Methyl-2-(2H-1,2,3-triazol-2-yl)benzoic acid (0.15 g, 0.72 mmol) and HATU (0.33 g, 0.86 mmol) were added to the reaction solution, and the mixture was stirred at the same temperature as above for 15 hours. Water and brine were added to the reaction solution, followed by extraction with EtOAc. The organic layer was washed with water and brine. The organic layer was dried over Na₂SO₄, and the desiccant was filtered off. Then, the solvent was distilled off under reduced pressure. A mixed solvent of EtOAc and Et₂O was added to the obtained residue, and the deposited solid was collected by filtration to obtain the title compound (0.17 g) (colorless solid).
LCMS retention time 3.84 min. (Condition 1)
MS (ESI pos.) m/z: 392 [M+H]+

### Example 2 N-{2-[4-(5-fluoropyridin-2-yl)-1H-pyrazol-1-yl]ethyl}-N,5-dimethyl-2-(2H-1,2,3-triazol-2-yl)benzamide

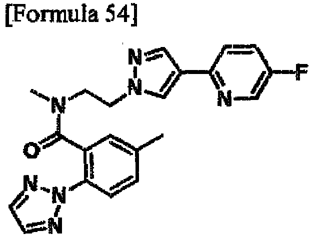

60% NaH (12 mg, 0.28 mmol) was added to a solution of the compound (0.090 g, 0.23 mmol) obtained in Example 1 in DMF (0.7 mL) at room temperature, and the mixture was stirred for 5 minutes. MeI (0.016 mL, 0.25 mmol) was added thereto at the same temperature as above, and the mixture was stirred overnight at room temperature. Water was added to the reaction solution, followed by extraction with EtOAc. The organic layer was dried over Na₂SO₄, and the desiccant was filtered off. Then, the solvent was distilled off under reduced pressure. The obtained residue was purified by column chromatography (KP-NH 11 g, hexane/EtOAc = 80/20 → 0/100). The obtained solid was washed with Et₂O with stirring and then filtered to obtain the title compound (0.045 g) (colorless solid).
LCMS retention time 4.24 min. (Condition 1)
MS (ESI pos.) m/z: 406 [M+H]+

### Example 3-1 N-{2-[4-(4-fluorophenyl)-1H-pyrazol-1-yl]ethyl}-5-methyl-2-(2H-1,2,3-triazol-2-yl)benzamide

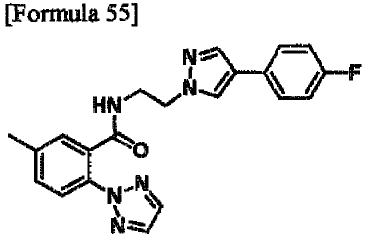

The title compound was obtained (colorless solid) by the same approach as in Example 1 using the compound obtained in Reference Example 23 and 5-methyl-2-(2H-1,2,3-triazol-2-yl)benzoic acid as starting materials.
MS (ESI pos.) m/z: 391 [M+H]+, 413 [M+Na]+

### Example 3-2 N-{2-[4-(4-fluorophenyl)-1H-pyrazol-1-yl]ethyl}-N,5-dimethyl-2-(2H-1,2,3-triazol-2-yl)benzamide

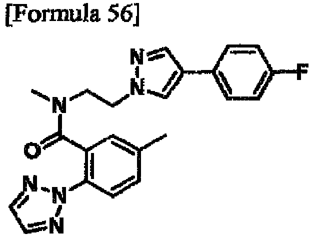

The title compound was obtained (colorless solid) by the same approach as in Example 2 using the compound obtained in Example 3-1 as a starting material.
LCMS retention time 5.19 min. (Condition 1)
MS (ESI pos.) m/z: 405 [M+H]+

### Example 4 N-ethyl-N-{2-[4-(5-fluoropyridin-2-yl)-1H-pyrazol-1-yl]ethyl}-5-methyl-2-(2H-1,2,3-triazol-2-yl)benzamide

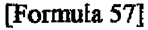

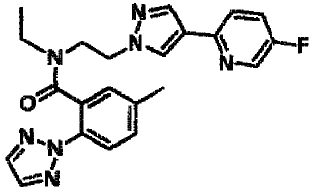

MsCl (0.10 mL, 1.3 mmol) was added to a solution of the compound (0.30 g, 1.1 mmol) obtained in Reference Example 10 and TEA (0.30 mL, 2.2 mmol) in CHCl₃ (6 mL) under cooling in ice water, and the mixture was heated to room temperature and stirred for 45 minutes. An aqueous NaHCO₃ solution was added thereto under cooling in ice water, followed by extraction with EtOAc. Then, the organic layer was washed with brine, dried over Na₂SO₄, and the desiccant was filtered off. Then, the solvent was distilled off under reduced pressure. Cs₂CO₃ (0.18 g, 0.56 mmol) was added to a suspension of the obtained residue and the compound (0.046 g, 0.28 mmol) obtained in Preference Example 5 in MeCN (0.56 mL), and the mixture was stirred in an oil bath with a temperature of 80°C for 1 hour. After standing to cool to room temperature, water was added thereto, followed by extraction with EtOAc. The solvent in the organic layer was distilled off under reduced pressure. The obtained residue was purified by column chromatography (HP-NH 11 g, hexane/EtOAc = 85/15 → 0/100). The obtained solid was washed with Et₂O with stirring and then collected by filtration to obtain the title compound (0.065 g) (colorless solid).
LCMS retention time 4.59 min. (Condition 1)
MS (ESI pos.) m/z: 420 [M+H]+

The compounds of Examples 5 to 14 were obtained using the same approach as in Example 4.

### Example 5 N-(cyclopropylmethyl)-N-{2-[4-(5-fluoropyridin-2-yl)-1H-pyrazol-1-yl]ethyl}-5-methyl-2-(2H-1,2,3-triazol-2-yl)benzamide

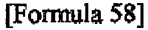

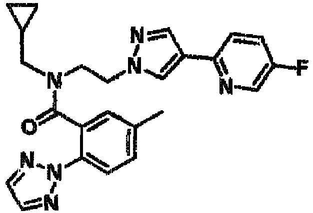

The title compound was obtained (colorless solid) using the compound obtained in Reference Example 5 and N-(cyclopropylmethyl)-N-(2-hydroxyethyl)-5-methyl-2-(2H-1,2,3-triazol-2-yl)benzamide obtained using the same approach as in Preference Example 10 as starting materials.
LCMS retention time 5.24 min. (Condition 1)
MS (ESI pos.) m/z: 446 [M+H]+

### Example 6 N-(cyclopropylmethyl)-N-{2-[4-(4-fluorophenyl)-1H-pyrazol-1-yl]ethyl}-5-methyl-2-(2H-1,2,3-triazol-2-yl)benzamide

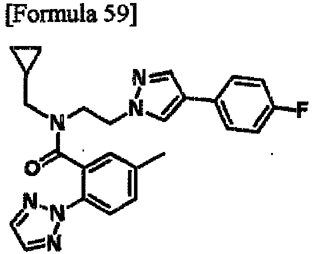

The title compound was obtained (colorless solid) using 4-(4-fluorophenyl)-1H-pyrazole and N-(cyclopropylmethyl)-N-(2-hydroxyethyl)-5-methyl-2-(2H-1,2,3-triazol-2-yl)benzamide as starting materials.
LCMS retention time 5.83 min. (Condition 1)
MS (ESI pos.) m/z: 445 [M+H]+

### Example 7 N-ethyl-N-{2-[4-(4-fluorophenyl)-1H-pyrazol-1-yl]ethyl}-5-methyl-2-(2H-1,2,3-triazol-2-yl)benzamide

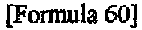

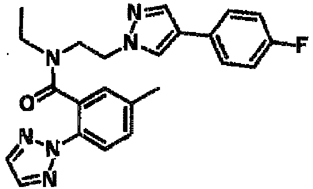

The title compound was obtained (colorless solid) using the compound obtained in Reference Example 10 and 4-(4-fluorophenyl)-1H-pyrazole as starting materials.
LCMS retention time 5.47 min. (Condition 1)
MS (ESI pos.) m/z: 419 [M+H]+

### Example 8 N-ethyl-N-{2-[3-(4-fluorophenyl)-1H-pyrazol-1-yl]ethyl}-5-methyl-2-(2H-1,2,3-triazol-2-yl)benzamide

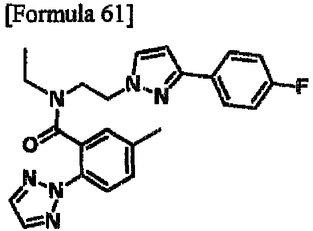

The title compound was obtained (colorless solid) using the compound obtained in Reference Example 10 and 3-(4-fluorophenyl)-1H-pyrazole as starting materials.
LCMS retention time 5.65 min. (Condition 1)
MS (ESI pos.) m/z: 419 [M+H]+

### Example 9 N-ethyl-5-methyl-N-{2-[3-(pyridin-2-yl)-1H-pyrazol-1-yl]ethyl}-2-(2H-1,2,3-triazol-2-yl)benzamide

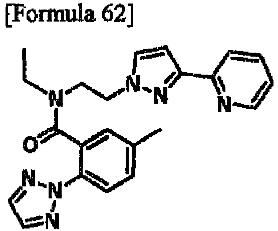

The title compound was obtained (colorless solid) using the compound obtained in Reference Example 10 and 2-(1H-pyrazol-3-yl)pyridine as starting materials.
LCMS retention time 3.37 min. (Condition 1)
MS (ESI pos.) m/z: 402 [M+H]+

### Example 10 N-{2-[4-(5-chloropyridin-2-yl)-1H-pyrazol-1-yl]ethyl}-N-ethyl-5-methyl-2-(2H-1,2,3-triazol-2-yl)benzamide

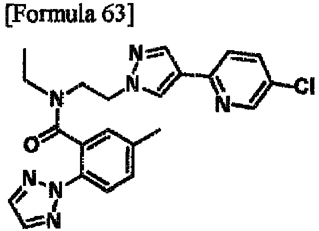

The title compound was obtained (colorless solid) using the compound obtained in Reference Example 10 and 5-chloro-2-(1H-pyrazol-4-yl)pyridine as starting materials.
LCMS retention time 5.03 min. (Condition 1)
MS (ESI pos.) m/z: 436 [M+H]+

### Example 11 N-ethyl-5-methyl-N-{2-[4-(pyridin-2-yl)-1H-pyrazol-1-yl]ethyl}-2-(2H-1,2,3-triazol-2-yl)benzamide

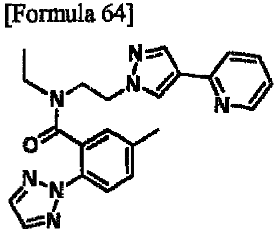

The title compound was obtained (colorless solid) using the compound obtained in Reference Example 10 and 2-(1H-pyrazol-4-yl)pyridine as starting materials.
LCMS retention time 3.30 min. (Condition 1)
MS (ESI pos.) m/z: 4.02 [M+H]+

### Example 12 N-ethyl-N-{2-[4-(6-methylpyridin-2-yl)-1H-pyrazol-1-yl]ethyl}-5-methyl-2-(2H-1,2,3-triazol-2-yl)benzamide

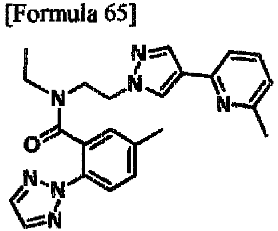

The title compound was obtained (colorless solid) using the compound obtained in Reference Example 10 and 2-methyl-6-(1H-pyrazol-4-yl)pyridine as starting material.
LCMS retention time 3.35 min. (Condition 1)
MS (ESI pos.) m/z: 416 [M+H]+

### Example 13 N-cyclopropyl-N-{2-[4-(5-fluoropyridin-2-yl)-1H-pyrazol-1-yl]ethyl}-5-methyl-2-(2H-1,2,3-triazol-2-yl)benzamide

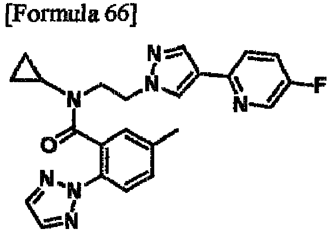

The title compound was obtained (colorless solid) using the compounds obtained in Reference Examples 13 and 5 as starting materials.
LCMS retention time 4.71 min. (Condition 1)
MS (ESI pos.) m/z: 432 [M+H]+

### Example 14 N-{2-[4-(5-fluoropyridin-2-yl)-1H-pyrazol-1-yl]ethyl}-5-methyl-N-(propan-2-yl)-2-(2H-1,2,3-triazol-2-yl)benzamide

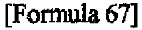

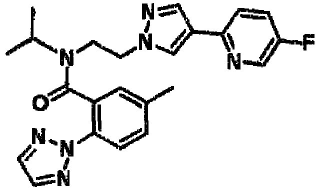

The title compound was obtained (colorless solid) using the compounds obtained in Reference Examples 25 and 5 as starting materials.
LCMS retention time 4.98 min. (Condition 1)
MS (ESI pos.) m/z : 434 [M+H]+

### Example 15 N-ethyl-N-{2-[4-(5-fluoropyridin-2-yl)-1H-pyrazol-1-yl]ethyl}-5-methyl-2-(pyrimidin-2-yl)benzamide

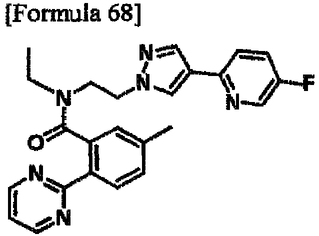

DIPEA (0.28 mL, 1.6 mmol) was added to a solution of the compound (0.15 g, 0.64 mmol) obtained in Reference Example 2 in CHCl₃ (3 mL) at room temperature. 5-Methyl-2-(pyrimidin-2-yl)benzoic acid (0.18 g, 0.83 mmol) and propylphosphonic acid anhydride (cyclic trimer) (50% solution in EtOAc (approximately 1.7 mol/L), 1.1 mL, 1.9 mmol) were added to the reaction solution under cooling in ice water. The resultant mixture was stirred at room temperature for 1 hour and further stirred in an oil bath with a temperature of 50°C for 3 hours. After standing to cool to room temperature, water was added thereto, followed by extraction with CHCl₃. The organic layer was washed with brine. Then, the organic layer was dried over Na₂SO₄, and the desiccant was filtered off. Then, the solvent was distilled off under reduced pressure. The obtained residue was purified by column chromatography (KP-NH 28 g, hexane/EtOAc = 70/30 → 0/100) and further purified by HPLC to obtain the title compound (0.069 g) (colorless amorphous).
LCMS retention time 4.37 min. (Condition 1)
MS (ESI pos.) m/z : 431 [M+H]+

### Example 16 N-{2-[3-(5-fluoropyridin-2-yl)-1H-pyrazol-1-yl]ethyl}-5-methyl-2-(2H-1,2,3-triazol-2-yl)benzamide

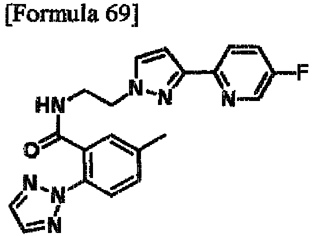

The title compound was obtained (colorless solid) by the same approach as in Example 1 using the compound obtained in Reference Example 4 and 5-methyl-2-(2H-1,2,3-triazol-2-yl)benzoic acid as starting materials.
LCMS retention time 4.07 min. (Condition 1)
MS (ESI pos.) m/z : 392 [M+H]+

### Example 17 N-{2-[3-(5-fluoropyridin-2-yl)-1H-pyrazol-1-yl]ethyl}-N,5-dimethyl-2-(2H-1,2,3-triazol-2-yl)benzamide

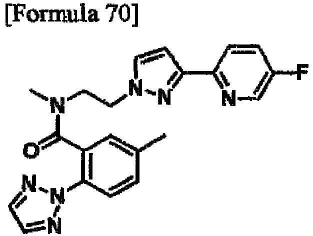

The title compound was obtained (colorless solid) by the same approach as in Example 2 using the compound obtained in Example 16 and MeI as starting materials.
LCMS retention time 4.50 min. (Condition 1)
MS (ESI pos.) m/z : 406 [M+H]+

The compounds of Examples 18, 19, 20-2, 21, 22, 23-2, and 24 were obtained using the same approach as in Example 17.

### Example 18 N-(cyclopropylmethyl)-N-{2-[3-(5-fluoropyridin-2-yl)-1H-pyrazol-1-yl]ethyl}-5-methyl-2-(2H-1,2,3-triazol-2-yl)benzamide

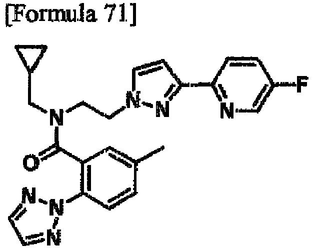

The title compound was obtained (colorless solid) using the compound obtained in Example 16 and cyclopropylmethyl bromide as starting materials.
LCMS retention time 5.23 min. (Condition 1)
MS (ESI pos.) m/z : 446 [M+H]+, 468 [M+Na]+

### Example 19 N-ethyl-N-{2-[3-(5-fluoropyridin-2-yl)-1H-pyrazol-1-yl]ethyl}-5-methyl-2-(2H-1,2,3-triazol-2-yl)benzamide

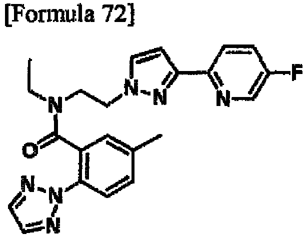

The title compound was obtained (colorless solid) using the compound obtained in Example 16 and EtI as starting materials.
LCMS retention time 4.78 min. (Condition 1)
MS (ESI pos.) m/z : 420 [M+H]+

### Example 20-1 N-{2-[3-(4-fluorophenyl)-1H-pyrazol-1-yl]ethyl}-5-methyl-2-(2H-1,2,3-triazol-2-yl)benzamide

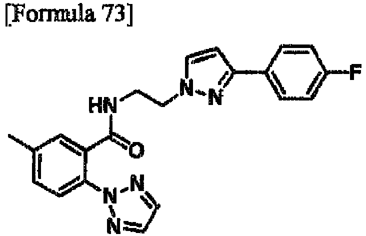

The title compound was obtained (colorless oil) by the same approach as in Example 1 using 2-[3-(4-fluorophenyl)-1H-pyrazol-1-yl]ethanamine dihydrochloride and 5-methyl-2-(2H-1,2,3-triazol-2-yl)benzoic acid as starting materials.
MS (ESI pos.) m/z : 391 [M+H]+

### Example 20-2 N-{2-[3-(4-fluorophenyl)-1H-pyrazol-1-yl]ethyl}-N,5-dimethyl-2-(2H-1,2,3-triazol-2-yl)benzamide

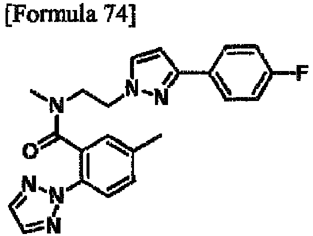

The title compound was obtained (colorless solid) using the compound obtained in Example 20-1 and MeI as starting materials.
LCMS retention time 5.37 min. (Condition 1)
MS (ESI pos.) m/z : 405 [M+H]+

### Example 21 N-{2-[3-(5-fluoropyridin-2-yl)-1H-pyrazol-1-yl]ethyl}-5-methyl-N-(2-fluoroethyl)-2-(2H-1,2,3-triazol-2-yl)benzamide

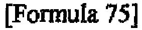

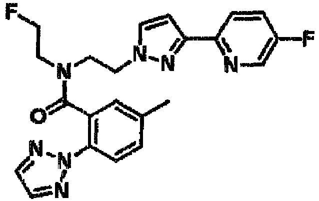

The title compound was obtained (colorless solid) using the compound obtained in Example 16 and 1-fluoro-2-iodoethane as starting materials.
LCMS retention time 4.71 min. (Condition 1)
MS (ESI pos.) m/z : 438 [M+H]+

### Example 22 N-(2,2-difluoroethyl)-N-{2-[3-(5-fluoropyridin-2-yl)-1H-pyrazol-1-yl]ethyl}-5-methyl-2-(2H-1,2,3-triazol-2-yl)benzamide

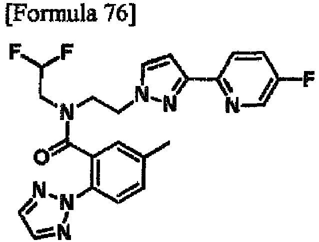

The title compound was obtained (colorless solid) using the compound obtained in Example 16 and 2,2-difluoroethyl trifluoromethanesulfonate as starting materials.
LCMS retention time 5.08 min. (Condition 1)
MS (ESI pos.) m/z : 456 [M+H]+

### Example 23-1 5-Fluoro-N-{2-[3-(5-fluoropyridin-2-yl)-1H-pyrazol-1-yl]ethyl}-2-(2H-1,2,3-triazol-2-yl)benzamide

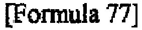

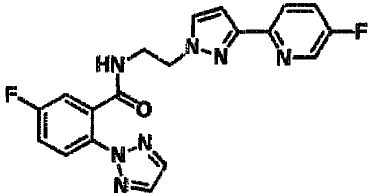

The title compound was obtained (brown powder) by the same approach as in Example 1 using the compound obtained in Reference Example 4 and 5-fluoro-2-(2H-1,2,3-triazol-2-yl)benzoic acid as starting materials.
MS (ESI pos.) m/z : 396[M+H]+

### Example 23-2 N-methyl-N-{2-[3-(5-fluoropyridin-2-yl)-1H-pyrazol-1-yl]ethyl}-5-fluoro-2-(2H-1,2,3-triazol-2-yl)benzamide

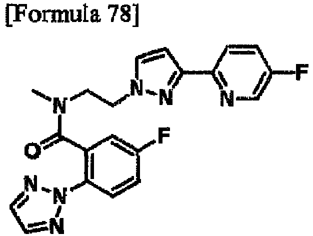

The title compound was obtained (colorless solid) using the compound obtained in Example 23-1 and MeI as starting materials.
LCMS retention time 4.37 min. (Condition 1)
MS (ESI pos.) m/z : 410 [M+H]+

### Example 24 N-ethyl-N-{2-[3-(5-fluoropyridin-2-yl)-1H-pyrazol-1-yl]ethyl}-5-fluoro-2-(2H-1,2,3-triazol-2-yl)benzamide

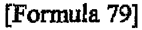

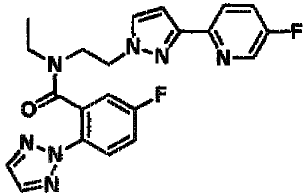

The title compound was obtained (colorless solid) using the compound obtained in Example 23-1 and EtI as starting materials.
LCMS retention time 4.64 min. (Condition 1)
MS (ESI pos.) m/z : 424 [M+H]+

### Example 25 N-{2-[3-(5-fluoropyridin-2-yl)-1H-pyrazol-1-yl]ethyl}-5-methyl-2-(pyrimidin-2-yl)benzamide

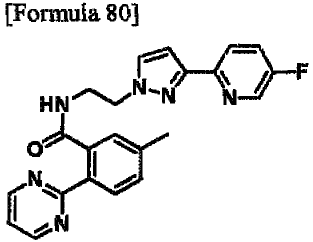

The title compound (0.093 g) was obtained (colorless solid) in the same approach as in Example 15 using the compound (0.20 g, 0.72 mmol) obtained in Reference Example 7 and 5-methyl-2-(pyrimidin-2-yl)benzoic acid (0.20 g, 0.93 mmol) as starting materials.
MS (ESI pos.) m/z : 403 [M+H]+

### Example 26 N-ethyl-N-{2-[3-(5-fluoropyridin-2-yl)-1H-pyrazol-1-yl]ethyl}-5-methyl-2-(pyrimidin-2-yl)benzamide

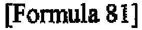

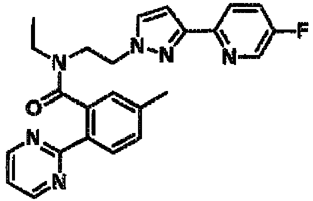

The title compound (0.055 g) was obtained (colorless solid) by the same approach as in Example 2 using the compound (0.093 g, 0.23 mmol) obtained in Example 25 and EtI (0.020 mL, 0.25 mmol) as starting materials.
LCMS retention time 4.63 min. (Condition 1)
MS (ESI pos.) m/z : 431 [M+H]+

The compounds of Examples 27 to 33 were obtained using the same approach as in Example 1.

### Example 27 N-{2-[3-(5-fluoropyridin-2-yl)-1H-pyrazol-1-yl]ethyl}-5-methyl-N-(propan-2-yl)-2-(2H-1,2,3-triazol-2-yl)benzamide

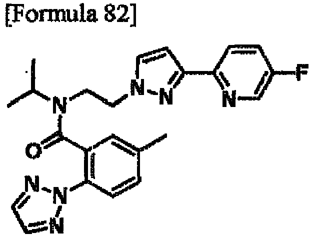

The title compound was obtained (colorless amorphous) using the compound obtained in Reference Example 16 and 5-methyl-2-(2H-1,2,3-triazol-2-yl)benzoic acid as starting material s.
LCMS retention time 5.24 min. (Condition 1)
MS (ESI pos.) m/z : 434 [M+H]+

### Example 28 N-(cyclopropylmethyl)-N-{2-[3-(4-fluorophenyl)-1H-pyrazol-1-yl]ethyl}-5-methyl-2-(2H-1,2,3-triazol-2-yl)benzamide

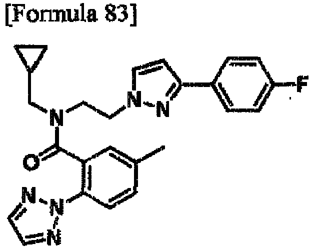

The title compound was obtained (colorless solid) using the compound obtained in Reference Example 26 and 5-methyl-2-(2H-1,2,3-triazol-2-yl)benzoic acid as starting materials.
LCMS retention time 6.04 min. (Condition 1)
MS (ESI pos.) m/z : 445 [M+H]+

### Example 29 N-(cyclopropylmethyl)-5-methyl-N-[2-(3-phenyl-1H-pyrazol-1-yl)ethyl]-2-(2H-1,2,3-triazol-2-yl)benzamide

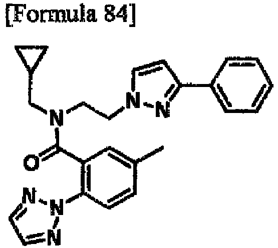

The title compound was obtained (colorless amorphous) using the compound obtained in Reference Example 27 and 5-methyl-2-(2H-1,2,3-triazol-2-yl)benzoic acid as starting materials.
LCMS retention time 5.95 min. (Condition 1)
MS (ESI pos.) m/z : 427 [M+H]+

### Example 30 N-cyclopropyl-N-{2-[3-(5-fluoropyridin-2-yl)-1H-pyrazol-1-yl]ethyl}-5-methyl-2-(2H-1,2,3-triazol-2-yl)benzamide

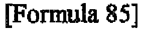

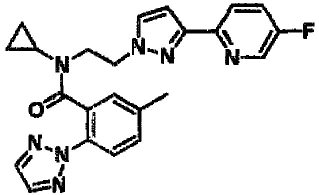

The title compound was obtained (colorless powder) using the compound obtained in Reference Example 17 and 5-methyl-2-(2H-1,2,3-triazol-2-yl)benzoic acid as starting materials.
LCMS retention time 4.95 min. (Condition 1)
MS (ESI pos.) m/z : 432 [M+H]+

### Example 31 N-{2-[3-(5-fluoropyridin-2-yl)-1H-pyrazol-1-yl]ethyl}-5-methyl-N-(oxetan-3-yl)-2-(2H-1,2,3-triazol-2-yl)benzamide

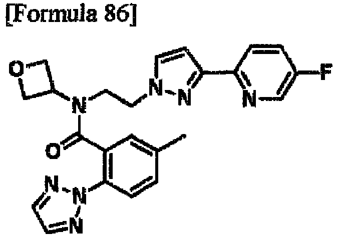

The title compound was obtained (colorless powder) using the compound obtained in Reference Example 28 and 5-methyl-2-(2H-1,2,3-triazol-2-yl)benzoic acid as starting materials.
LCMS retention time 4.43 min. (Condition 1)
MS (ESI pos.) m/z : 448 [M+H]+

### Example 32 N-ethyl-N-{2-[3-(5-fluoropyridin-2-yl)-1H-pyrazol-1-yl]ethyl}-2-(2H-1,2,3-triazol-2-yl)benzamide

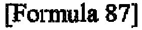

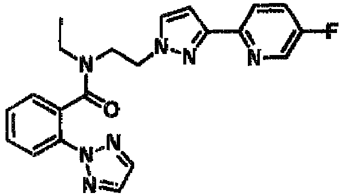

The title compound was obtained (colorless solid) using the compound obtained in Reference Example 15-1 and 2-(2H-1,2,3-triazol-2-yl)benzoic acid as a starting material.
LCMS retention time 4.56 min. (Condition 1)
MS (ESI pos.) m/z : 406 [M+H]+

### Example 33 5-Chloro-N-ethyl-N-{2-[3-(5-fluoropyridin-2-yl)-1H-pyrazol-1-yl]ethyl}-2-(2H-1,2,3-triazol-2-yl)benzamide

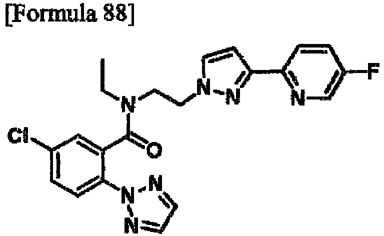

The title compound was obtained (colorless solid) using the compound obtained in Reference Example 15-1 and 5-chloro-2-(2H-1,2,3-triazol-2-yl)benzoic acid as starting materials.
LCMS retention time 5.03 min. (Condition 1)
MS (ESI pos.) m/z : 440 [M+H]+

### Example 34 N-ethyl-N-{2-[3-(5-fluoropyridin-2-yl)-1H-pyrazol-1-yl]ethyl}-2-(pyrimidin-2-yl)benzamide

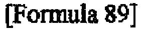

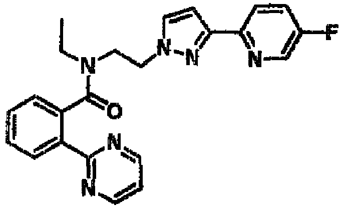

The title compound was obtained (colorless solid) by the same approach as in Example 15 using the compound obtained in Reference Example 15-1 and 2-(pyrimidin-2-yl)benzoic acid as starting materials.
LCMS retention time 4.32 min. (Condition 1)
MS (ESI pos.) m/z: 417 [M+H]+

### Example 35 N-ethyl-N-{2-[3-(5-fluoropyridin-2-yl)-1H-pyrazol-1-yl]ethyl}-5-methyl-2-(pyridin-2-yl)benzamide

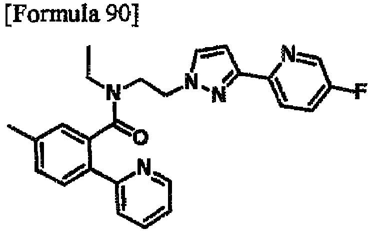

Pd(PPh₃)₄ (0.028 g, 0.020 mmol), copper iodide (0.011 g, 0.030 mmol), and cesium fluoride (0.11 g, 0.71 mmol) were added to a solution of the compound (0.17 g, 0.36 mmol) obtained in Reference Example 21 and 2-(tributylstannanyl)pyridine (0.17 g, 0.46 mmol) in toluene (9 mL), and the mixture was stirred in an oil bath with a temperature of 110°C for 15 hours. Brine was added to the reaction mixture, followed by extraction with EtOAc. The organic layer was dried over Na₂SO₄, and the desiccant was filtered off. Then, the solvent was distilled off under reduced pressure. The obtained residue was purified by column chromatography (KP-NH 28 g, hexane/EtOAc = 70/30 → 0/100) and further purified by HPLC. Et₂O was added to the obtained residue, and the deposited solid was collected by filtration to obtain the title compound (0.048 g) (colorless solid).
LCMS retention time 3.44 min. (Condition 1)
MS (ESI pos.) m/z : 430 [M+H]+

### Example 36 N-ethyl-N-{2-[3-(5-fluoropyridin-2-yl)-1H-pyrazol-1-yl]ethyl}-5-methyl-2-(pyridin-3-yl)benzamide

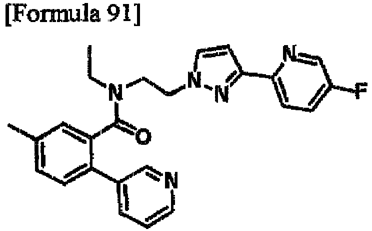

A 2 mol/L aqueous Na₂CO₃ solution (1.0 mL, 2.1 mmol) and Pd(PPh₃)₄ (0.026 g, 0.020 mmol) were added to a solution of the compound (0.17 g, 0.34 mmol) obtained in Reference Example 21 and pyridin-3-ylboronic acid (0.13 g, 1.1 mmol) in 1,4-dioxane (0.7 mL), and the mixture was stirred in an oil bath with a temperature of 110°C for 15 hours. After standing to cool to room temperature, brine was added to the reaction mixture, followed by extraction with EtOAc. The organic layer was dried over Na₂SO₄, and the desiccant was filtered off. Then, the solvent was distilled off under reduced pressure. The obtained residue was purified by column chromatography (KP-NH 28 g, hexane/EtOAc = 70/30 → 0/100) and further purified by HPLC. Et₂O was added to the obtained residue, and the deposited solid was collected by filtration to obtain the title compound (0.10 g) (colorless solid).
LCMS retention time 3.24 min. (Condition 1)
MS (ESI pos.) m/z : 430 [M+H]+

### Example 37 N-ethyl-N-{2-[4-(5-fluoropyridin-2-yl)-1H-pyrazol-1-yl]ethyl}-2-(pyrimidin-2-yl)benzamide

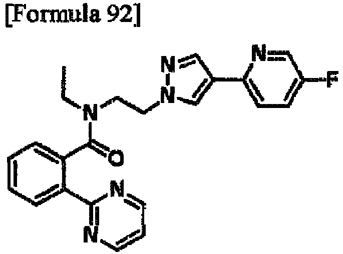

The title compound was obtained (colorless amorphous) by the same approach as in Example 15 using the compound obtained in Reference Example 19 and 2-(pyrimidin-2-yl)benzoic acid as starting materials.
LCMS retention time 4.14 min. (Condition 1)
MS (ESI pos.) m/z : 417 [M+H]+

### Example 38 5-Methyl-N-{2-[3-(6-methylpyridin-2-yl)-1H-pyrazol-1-yl]ethyl}-2-(2H-1,2,3-triazol-2-yl)benzamide

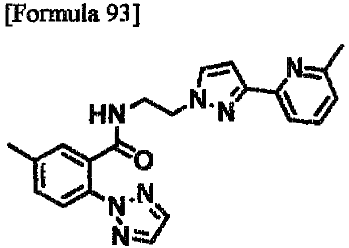

The title compound (0.090 g) was obtained by the same approach as in Example 1 using the compound (0.079 g, 0.39 mmol) obtained in Reference Example 30 and 5-methyl-2-(2H-1,2,3-triazol-2-yl)benzoic acid (0.13 g, 0.47 mmol) as starting materials.
MS (ESI pos.) m/z : 388 [M+H]+

### Example 39 N-ethyl-5-methyl-N-{2-[3-(6-methylpyridin-2-yl)-1H-pyrazol-1-yl]ethyl}-2-(2H-1,2,3-triazol-2-yl)benzamide

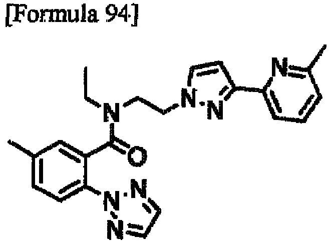

The title compound (0.090 g) was obtained by the same approach as in Example 2 using the compound (0.079 g, 0.23 mmol) obtained in Example 38 and ethyl iodide (0.022 ml, 0.28 mmol) as starting materials.
LCMS retention time 3.49 min. (Condition 2)
MS (ESI pos.) m/z : 416 [M+H]+

### Example 40 5-Fluoro-N-{2-[3-(5-fluoropyridin-2-yl)-1H-pyrazol-1-yl]ethyl}-2-(pyrimidin-2-yl)benzamide

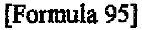

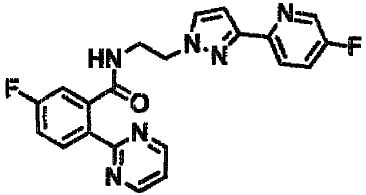

The title compound (0.040 g) was obtained by the same approach as in Example 15 using the compound (0.20 g, 0.72 mmol) obtained in Reference Example 4 and 5-fluoro-2-(pyrimidin-2-yl)benzoic acid (0.20 g, 0.93 mmol) as starting materials.
MS (ESI pos.) m/z : 407 [M+H]+

### Example 41 N-ethyl-5-fluoro-N-{2-[3-(5-fluoropyridin-2-yl)-1H-pyrazol-1-yl]ethyl}-2-(pyrimidin-2-yl)benzamide

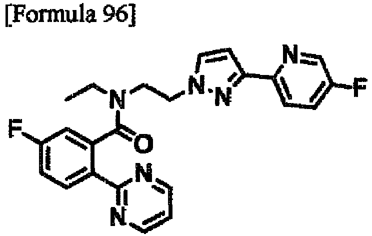

The title compound (0.034 g) was obtained by the same approach as in Example 2 using the compound (0.040 g, 0.10 mmol) obtained in Example 40 and ethyl iodide (0.008 ml, 0.10 mmol) as starting materials.
LCMS retention time 0.89 min. (Condition 2)
MS (ESI pos.) m/z : 435 [M+H]+

### Example 42 5-Chloro-N-{2-[3-(5-fluoropyridin-2-yl)-1H-pyrazol-1-yl]ethyl}-2-(pyrimidin-2-yl)benzamide

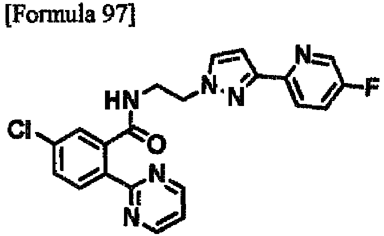

The title compound (0.037 g) was obtained by the same approach as in Example 15 using the compound (0.20 g, 0.72 mmol) obtained in Reference Example 4 and 5-chloro-2-(pyrimidin-2-yl)benzoic acid (0.22 g, 0.93 mmol) as starting materials.
MS (ESI pos.) m/z : 423 [M+H]+

### Example 43 N-ethyl-5-chloro-N-{2-[3-(5-fluoropyridin-2-yl)-1H-pyrazol-1-yl]ethyl}-2-(pyrimidin-2-yl)benzamide

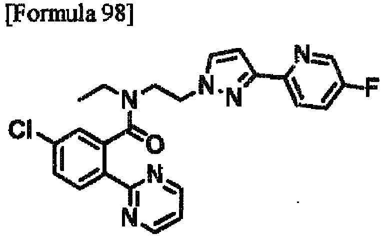

The title compound (0.025 g) was obtained by the same approach as in Example 2 using the compound (0.037 g, 0.09 mmol) obtained in Example 42 and ethyl iodide (0.007 ml, 0.09 mmol) as starting materials.
LCMS retention time 0.96 min. (Condition 2)
MS (ESI pos.) m/z : 451 [M+H]+

### Example 44 N-ethyl-4-fluoro-N-{2-[3-(5-fluoropyridin-2-yl)-1H-pyrazol-1-yl]ethyl}-2-(2H-1,2,3-triazol-2-yl)benzamide

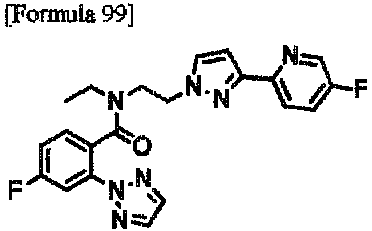

The title compound (0.040 g) was obtained by the same approach as in Example 1 using the compound (0.020 g, 0.09 mmol) obtained in Reference Example 15-2 and 4-fluoro-2-(2H-1,2,3-triazol-2-yl)benzoic acid (0.021 g, 0.10 mmol) as starting materials.
LCMS retention time 0.91 min. (Condition 2)
MS (ESI pos.) m/z : 424[M+H]+

### Example 45 N-ethyl-2-fluoro-N-{2-[3-(5-fluoropyridin-2-yl)-1H-pyrazol-1-yl]ethyl}-6-(2H-1,2,3-triazol-2-yl)benzamide

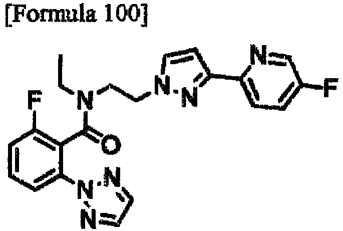

The title compound (0.021 g) was obtained by the same approach as in Example 1 using the compound (0.059 g, 0.19 mmol) obtained in Reference Example 15-2 and 2-fluoro-6-(2H-1,2,3-triazol-2-yl)benzoic acid (0.040 g, 0.19 mmol) as starting materials.
LCMS retention time 0.84, 0.92 min. (Condition 2)
MS (ESI pos.) m/z : 424[M+H]+

### Example 46 N-ethyl-2-fluoro-N-{2-[3-(5-fluoropyridin-2-yl-1H-pyrazol-1-yl]ethyl}-6-(pyrimidin-2-yl)benzamide

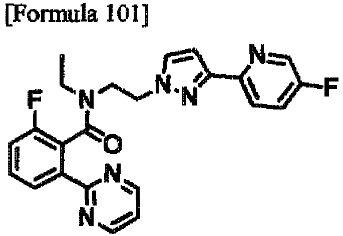

The title compound (0.008 g) was obtained by the same approach as in Example 15 using the compound (0.040 g, 0.14 mmol) obtained in Reference Example 15-2 and 2-fluoro-6-(pyrimidin-2-yl)benzoic acid (0.034 g, 0.16 mmol) as starting materials.
LCMS retention time 0.82, 0.91 min. (Condition 2)
MS (ESI pos.) m/z : 435[M+H]+

### Example 47 N-ethyl-N-{2-[3-(5-fluoropyridin-2-yl)-1H-pyrazol-1-yl]ethyl}-4-methyl-2-(2H-1,2,3-triazol-2-yl)benzamide

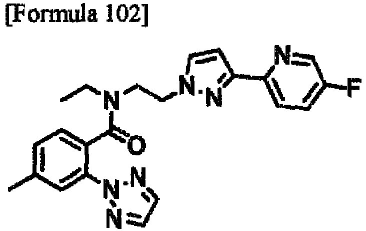

The title compound (0.036 g) was obtained by the same approach as in Reference Example 8 using the compound (0.050 g, 0.16 mmol) obtained in Reference Example 15-2 and 4-methyl-2-(2H-1,2,3-triazol-2-yl)benzoic acid (0.040 g, 0.20 mmol) as starting materials.
LCMS retention time 0.94 min. (Condition 2)
MS (ESI pos.) m/z : 420[M+H]+

### Example 48 N-ethyl-3-fluoro-N-{2-[3-(5-fluoropyridin-2-yl)-1H-pyrazol-1-yl]ethyl}-2-(2H-1,2,3-triazol-2-yl)benzamide

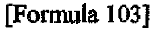

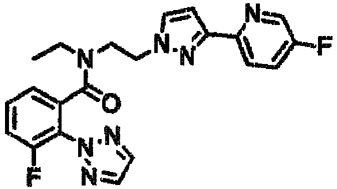

The title compound (0.029 g) was obtained by the same approach as in Reference Example 8 using the compound (0.030 g, 0.10 mmol) obtained in Reference Example 15-2 and 3-fluoro-2-(2H-1,2,3-triazol-2-yl)benzoic acid (0.024 g, 0.12 mmol) as starting materials.
LCMS retention time 0.84 min. (Condition 2)
MS (ESI pos.) m/z : 424[M+H]+

### Example 49 N-ethyl-4-fluoro-N-{2-[3-(5-fluoropyridin-2-yl)-1H-pyrazol-1-yl]ethyl}-2-(pyrimidin-2-yl)benzamide

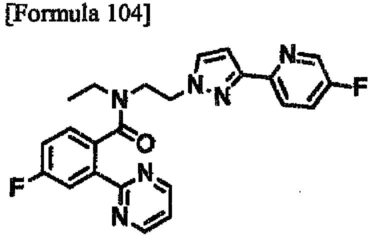

The title compound (0.028 g) was obtained by the same approach as in Example 15 using the compound (0.050 g, 0.16 mmol) obtained in Reference Example 15-2 and 4-fluoro-2-(pyrimidin-2-yl)benzoic acid (0.046 g, 0.21 mmol) as starting materials.
LCMS retention time 0.89 min. (Condition 2)
MS (ESI pos.) m/z : 435[M+H]+

### Example 50 N-ethyl-N-{2-[3-(5-fluoropyridin-2-yl)-1H-pyrazol-1-yl]ethyl}-5-methoxy-2-(2H-1,2,3-triazol-2-yl)benzamide

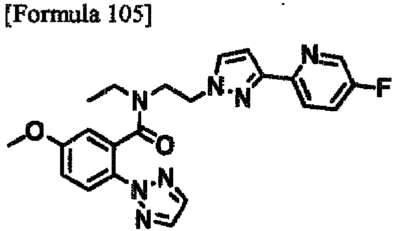

The title compound (0.013 g) was obtained by the same approach as in Example 1 using the compound (0.060 g, 0.20 mmol) obtained in Reference Example 15-2 and 5-methoxy-2-(2H-1,2,3-triazol-2-yl)benzoic acid (0.070 g, 0.30 mmol) as starting materials.
LCMS retention time 0.76 min. (Condition 2)
MS (ESI pos.) m/z: 436[M+H]+

### Example 51 N-ethyl-5-methyl-2-(2H-1,2,3-triazol-2-yl)-N-(2-{3-[5-(trifluoromethyl)pyridin-2-yl]-1H-pyrazol-1-yl}ethyl)benzamide

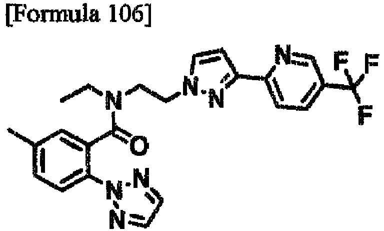

The title compound (0.026 g) was obtained by the same approach as in Reference Example 8 using the compound (0.030 g, 0.11 mmol) obtained in Reference Example 33 and 5-methyl-2-(2H-1,2,3-triazol-2-yl)benzoic acid (0.026 g, 0.13 mmol) as starting materials.
LCMS retention time 1.07 min. (Condition 2)
MS (ESI pos.) m/z : 470[M+H]+

### Example 52 N-ethyl-5-methyl-2-(2H-1,2,3-triazol-2-yl)-N-(2-{3-[6-(trifluoromethyl)pyridin-2-yl]-1H-pyrazol-1-yl}ethyl)benzamide

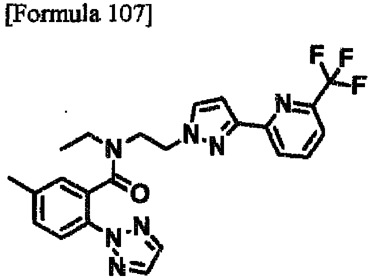

The title compound (0.010 g) was obtained by the same approach as in Example 1 using the compound (0.050 g, 0.16 mmol) obtained in Reference Example 36 and 5-methyl-2-(2H-1,2,3-triazol-2-yl)benzoic acid (0.050 g, 0.23 mmol) as starting materials.
LCMS retention time 1.09 min. (Condition 2)
MS (ESI pos.) m/z : 492[M+Na]+

### Example 53 N-{2-[3-(3,4-difluorophenyl)-1H-pyrazol-1-yl]ethyl}-N-ethyl-5-fluoro-2-(pyrimidin-2-yl)benzamide

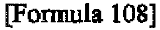

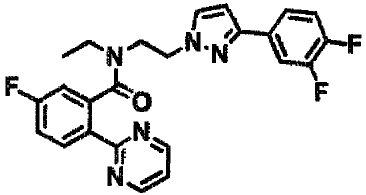

The title compound (0.009 g) was obtained by the same approach as in Example 15 using the compound (0.050 g, 0.17 mmol) obtained in Reference Example 37 and 5-fluoro-2-(pyrimidin-2-yl)benzoic acid (0.045 g, 0.21 mmol) as starting materials.
LCMS retention time 1.06 min. (Condition 2)
MS (ESI pos.) m/z : 452[M+H]+

### Example 54 N-ethyl-5-fluoro-N-{2-[3-(4-fluorophenyl)-1H-pyrazol-1-yl]ethyl}-2-(pyrimidin-2-yl)benzamide

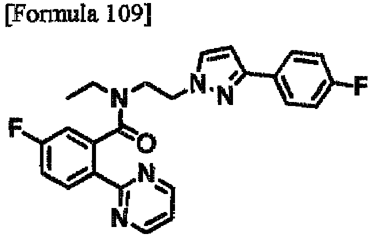

The title compound (0.050 g) was obtained by the same approach as in Example 15 using the compound (0.050 g, 0.19 mmol) obtained in Reference Example 38 and 5-fluoro-2-(pyrimidin-2-yl)benzoic acid (0.055 g, 0.24 mmol) as starting materials.
LCMS retention time 1.03 min. (Condition 2)
MS (ESI pos.) m/z : 434[M+H]+

### Example 55 5-Chloro-N-ethyl-N-{2-[4-(5-fluoropyridin-2-yl)-1H-pyrazol-1-yl]ethyl}-2-(2H-1,2,3-triazol-2-yl)benzamide

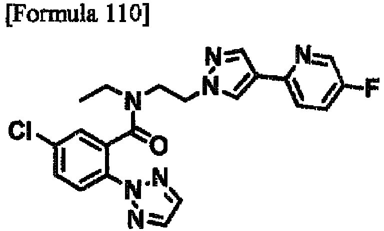

The title compound (0.093 g) was obtained by the same approach as in Example 1 using the compound (0.10 g, 0.43 mmol) obtained in Reference Example 19 and 5-chloro-2-(2H-1,2,3-triazol-2-yl)benzoic acid (0.14 g, 0.64 mmol) as starting materials.
LCMS retention time 0.95 min. (Condition 2)
MS (ESI pos.) m/z: 440[M+H]+

### Example 56 N-ethyl-5-fluoro-N-{2-[4-(5-fluoropyridin-2-yl)-1H-pyrazol-1-yl]ethyl}-2-(2H-1,2,3-triazol-2-yl)benzamide

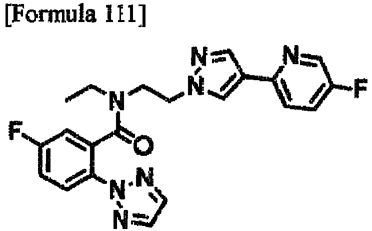

The title compound (0.099 g) was obtained by the same approach as in Example 1 using the compound (0.10 g, 0.43 mmol) obtained in Reference Example 19 and 5-fluoro-2-(2H-1,2,3-triazol-2-yl)benzoic acid (0.11 g, 0.51 mmol) as starting materials.
LCMS retention time 0.86 min. (condition 2)
MS (ESI pos.) m/z: 424[M+H]+

### Example 57 N-ethyl-5-fluoro-2-(pyrimidin-2-yl)-N-(2-{3-[6-(trifluoromethyl)pyridin-2-yl]-1H-pyrazol-1-yl}ethyl)benzamide

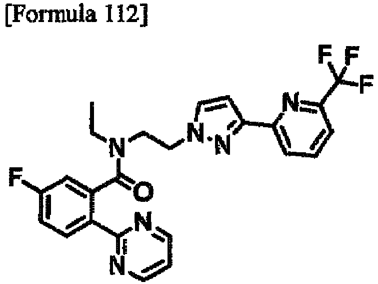

The title compound (0.008 g) was obtained by the same approach as in Example 15 using the compound (0.035 g, 0.11 mmol) obtained in Reference Example 36 and 4-fluoro-2-(pyrimidin-2-yl)benzoic acid (0.036 g, 0.17 mmol) as starting materials.
LCMS retention time 1.06 min. (Condition 2)
MS (ESI pos.) m/z: 485[M+H]+

### Example 58 N-ethyl-(pyrimidin-2-yl)-N-(2-{3-[6-(trifluoromethyl)pyridin-2-yl]-1H-pyrazol-1-yl}ethyl)benzamide

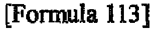

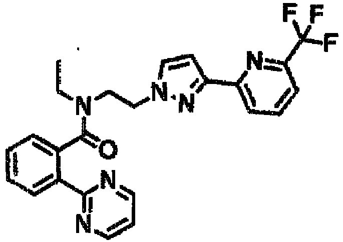

The title compound (0.039 g) was obtained by the same approach as in Example 15 using the compound (0.035 g, 0.11 mmol) obtained in Reference Example 36 and 2-(pyrimidin-2-yl)benzoic acid (0.035 g, 0.17 mmol) as starting materials.
LCMS retention time 1.03 min. (Condition 2)
MS (ESI pos.) m/z: 467[M+H]+

### Example 59 N-{2-[3-(3,4-difluorophenyl)-1H-pyrazol-1-yl]ethyl}-N-ethyl-2-(pyrimidin-2-yl)benzamide

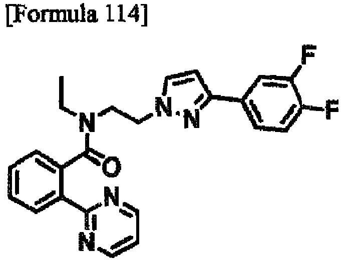

The title compound (0.060 g) was obtained by the same approach as in Example 15 using the compound (0.050 g, 0.17 mmol) obtained in Reference Example 37 and 2-(pyrimidin-2-yl)benzoic acid (0.050 g, 0.24 mmol) as starting materials.
LCMS retention time 1.04 min. (Condition 2)
MS (ESI pos.) m/z: 434[M+H]+

### Example 60 N-ethyl-5-methyl-2-(2H-1,2,3-triazol-2-yl)-N-(2-{3-[4-(trifluoromethyl)pyridin-2-yl]-1H-pyrazol-1-yl}ethyl)benzamide

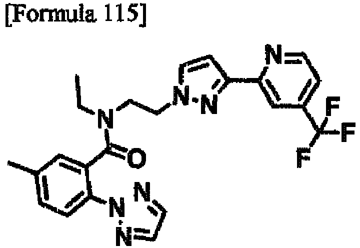

The title compound (0.082 g) was obtained by the same approach as in Example 1 using the compound (0.10 g, 0.28 mmol) obtained in Reference Example 41 and 5-methyl-2-(2H-1,2,3-triazol-2-yl)benzoic acid (0.068 g, 0.34 mmol) as starting materials.
LCMS retention time 1.06 min. (Condition 2)
MS (ESI pos.) m/z: 470[M+H]+

### Example 61 N-ethyl-5-fluoro-2-(pyrimidin-2-yl)-N-(2-{3-[4-(trifluoromethyl)pyridin-2-yl]-1H-pyrazol-1-yl}ethyl)benzamide

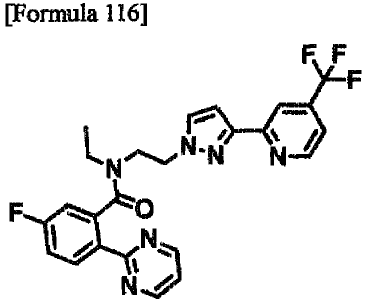

The title compound (0.13 g) was obtained by the same approach as in. Example 15 using the compound (0.10 g, 0.28 mmol) obtained in Reference Example 41 and 4-fluoro-2-(pyrimidin-2-yl)benzoic acid (0.073 g, 0.34 mmol) as starting materials.
LCMS retention time 1.02 min. (Condition 2)
MS (ESI pos.) m/z: 485[M+H]+

### Test Example (Determination of orexin receptor antagonistic activity)

The antagonistic activity of each test compound against human orexin type 1 receptor (hOX1R) and orexin type 2 receptor (hOX2R) was performed as described in the literature with a modification (Toshikatsu Okumura et al., Biochemical and Biophysical Research Communications 280, 976-981, 2001). Chinese hamster ovary (CHO) cells forced to express hOX1R or hOX2R were seeded at 20,000 cells/well on a 96 well Black clear bottom plates (Nunc) and cultured for 16 hours under conditions of 37°C and 5% CO₂ using a Ham's F-12 medium containing 0.1 mM MEM non-essential amino acids, 0.5 mg/ml G418, and 10% fetal bovine serum (all from Invitrogen Corp.). After removal of the medium, 100 µL of the assay buffer (25 mM HEPES (Dojindo Laboratories), Hanks' balanced salt solution (Invitrogen Corp.), 0.1% bovine serum albumin, 2.5 mM probenecid, 200 µg/ml Amaranth (all from Sigma-Aldrich Corp.), pH 7.4) containing 0.5 µM Fluo-4AM ester (Dojindo Laboratories) was added thereto, and the cells were incubated for 60 minutes under conditions of 37°C and 5% CO₂. After removal of the buffer solution for assay containing Fluo-4AM ester, the test compound was dissolved at a concentration of 10 mM in dimethyl sulfoxide and diluted with the assay buffer, and 150 µL of the diluted solution was then added to each wells, which were then incubated for 30 minutes.
A peptide (Pyr-Pro-Leu-Pro-Asp-Ala-Cys-Arg-Gln-Lys-Thr-Ala-Ser-Cys-Arg-Leu-Tyr-Glu-Leu-Leu-His-Gly-Ala-Gly-Asn-His-Ala-Ala-Gly-Ile-Leu-Thr-Leu-NH2; Peptide Institute, Inc.) substituted 2 amino acids of human orexin-A was diluted with the assay buffer to 300 pM (final concentration for hOX1R) or 3 nM (final concentration for hOX2R). 50 µL of this ligand solution was added thereto to start reaction. During the reaction, the fluorescence value of each well was measured every one second for 3 minutes using Functional Drug Screening System (FDSS; manufactured by Hamamatsu Photonics K.K.), and antagonistic activity was determined with the maximum fluorescence value as an index for intracellular Ca²⁺ concentration. The antagonistic activity of the test compound was calculated with the fluorescence value of a well supplemented with only a dilution buffer solution as 100% and the fluorescence value of a well supplemented with a buffer solution containing neither the ligand nor the compound as 0%, and determined in terms of 50% inhibitory concentration (IC₅₀ value) from fluorescence values derived from the test compound added at varying concentration.

The IC₅₀ values of the compounds of the present invention are shown in Table 1.
In this context, the compounds of Examples 3-1, 20-1, 23-1, and 25 were not subjected to the orexin receptor antagonistic activity test.

**[Table 1]**

| Example No. | IC₅₀ value | | Example No. | IC₅₀ value | |
|---|---|---|---|---|---|
| | O×1R (nM) | O×2R (nM) | | O×1R (nM) | O×2R (nM) |
| 1 | 1037.3 | 2387.5 | 31 | 24.9 | 26.9 |
| 2 | 17.9 | 47.3 | 32 | 9.0 | 6.6 |
| 3-2 | 2.2 | 7.1 | 33 | 6.5 | 2.2 |
| 4 | 6.0 | 14.6 | 34 | 8.4 | 8.9 |
| 5 | 102.3 | 38.7 | 35 | 1.8 | 11.7 |
| 6 | 50.1 | 2.6 | 36 | 30.6 | 58.9 |
| 7 | 2.3 | 6.3 | 37 | 109.6 | 301.2 |
| 8 | 0.9 | 2.5 | 39 | 1.5 | 2.2 |
| 9 | 1.0 | 4.3 | 41 | 0.5 | 7.6 |
| 10 | 14.4 | 52.6 | 43 | 1.8 | 3.5 |
| 11 | 10.5 | 17.2 | 44 | 17.3 | 29.8 |
| 12 | 1.8 | 22.7 | 45 | 38.7 | 46.9 |
| 13 | 9.8 | 15.4 | 46 | 92.5 | 149.2 |
| 14 | 596.9 | 412.4 | 47 | 0.8 | 7.1 |
| 15 | 29.8 | 88.3 | 48 | 5.5 | 10.3 |
| 16 | 115.2 | 598.2 | 49 | 7.1 | 48.8 |
| 17 | 5.6 | 22.8 | 50 | 20.0 | 213.7 |
| 18 | 6.8 | 2.2 | 51 | 50.9 | 393.9 |
| 19 | 1.5 | 3.9 | 52 | 1.1 | 1.3 |
| 20-2 | 0.9 | 8.8 | 53 | 1.5 | 7.8 |
| 21 | 3.7 | 20.3 | 54 | 2.0 | 19.2 |
| 22 | 10.5 | 32.5 | 55 | 1.6 | 2.3 |
| 23-2 | 76.0 | 132.5 | 56 | 38.0 | 95.9 |
| 24 | 7.2 | 12.2 | 57 | 22.9 | 99.5 |
| 26 | 1.4 | 3.7 | 58 | 78.9 | 140.1 |
| 27 | 20.9 | 43.6 | 59 | 1.9 | 5.6 |
| 28 | 1.2 | 4.0 | 60 | 21.9 | 127.0 |
| 29 | 1.5 | 6.1 | 61 | 239.0 | 1130.9 |
| 30 | 1.3 | 1.8 | | | |

### [Industrial Applicability]

The compound of the present invention has been shown to have an OX receptor antagonistic property. Thus, the compound of the present invention or the pharmaceutically acceptable salt thereof can be used as a therapeutic or prophylactic agent for diseases regulated by the OX receptor antagonistic effect, for example, sleep disorder, depression, anxiety disorder, panic disorder, schizophrenia, drug dependence, Alzheimer's disease, Parkinson's disease, Huntington's chorea, eating disorder, pain, gastrointestinal disease, epilepsy, inflammation, immune-related diseases, endocrine-related diseases, and hypertension.

## Claims

1. A pyrazole derivative represented by formula (IA) or a pharmaceutically acceptable salt thereof: 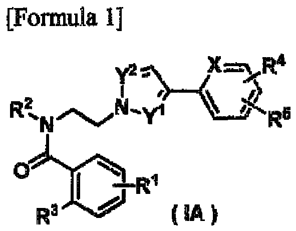 wherein
X represents a nitrogen atom or a formula CH;
any one of Y¹ and Y² represents a nitrogen atom, and the other of Y¹ and Y² represents a formula CH;
R¹ represents a hydrogen atom, a halogen atom, a C₁₋₆ alkyl group, or a C₁₋₆ alkoxy group;
R² represents a hydrogen atom, a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group,
wherein Substituent group 1 is a group consisting of a halogen atom, a C₃₋₆ cycloalkyl group, and a C₁₋₆ alkoxy group;
R³ represents a triazolyl group, a pyridyl group, or a pyrimidyl group,
wherein the triazolyl group, the pyridyl group, or the pyrimidyl group may be substituted with 1 to 3 halogen atoms; and
R⁴ and R⁵ are the same or different and each represent a hydrogen atom, a halogen atom, or a C₁₆ alkyl group, wherein the C₁₋₆ alkyl group may be substituted with 1 to 3 halogen atoms.

2. The pyrazole derivative or a pharmaceutically acceptable salt thereof according to claim 1, wherein R⁵ is a hydrogen atom.

3. A pyrazole derivative represented by formula (I) or a pharmaceutically acceptable salt thereof: 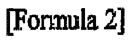 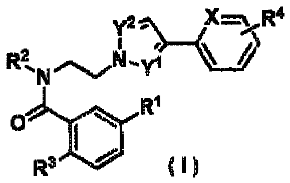 wherein
X represents a nitrogen atom or a formula CH;
any one of Y¹ and Y² represents a nitrogen atom, and the other of Y¹ and Y² represents a formula CH;
R¹ represents a hydrogen atom, a halogen atom, or a C₁₋₆ alkyl group;
R² represents a hydrogen atom, a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group,
wherein Substituent group 1 is a group consisting of a halogen atom, a C₃₋₆ cycloalkyl group, and a C₁₋₆ alkoxy group;
R³ represents a triazolyl group, a pyridyl group, or a pyrimidyl group,
wherein the triazolyl group, the pyridyl group, or the pyrimidyl group may be substituted with 1 to 3 halogen atoms; and
R⁴ represents a hydrogen atom, a halogen atom, or a C₁₋₆ alkyl group.

4. The pyrazole derivative or a pharmaceutically acceptable salt thereof according to any one of claims 1 to 3, wherein R² is a C₁₋₆ alkyl group, wherein the C₁₋₆ alkyl group may be substituted with 1 to 3 substituents selected from Substituent group 1, a C₃₋₆ cycloalkyl group, or a 4- to 6-membered cyclic ether group.

5. The pyrazole derivative or a pharmaceutically acceptable salt thereof according to any one of claims 1 to 4, wherein R² is a methyl group or an ethyl group.

6. The pyrazole derivative or a pharmaceutically acceptable salt thereof according to any one of claims 1 to 5, wherein
X is a nitrogen atom, and
R³ is a triazolyl group or a pyrimidyl group.

7. Any one or a mixture of two or more selected from the following group of the compounds and pharmaceutically acceptable salts thereof according to claim 1:
N-{2-[4-(5-fluoropyridin-2-yl)-1H-pyrazol-1-yl]ethyl}-5-methyl-2-(2H-1,2,3-triazol-2-yl)benzamide,
N-{2-[4-(5-fluoropyridin-2-yl)-1H-pyrazol-1-yl]ethyl}-N,5-dimethyl-2-(2H-1,2,3-triazol-2-yl)benzamide,
N-{2-[4-(4-fluorophenyl)-1H-pyrazol-1-yl]ethyl}-5-methyl-2-(2H-1,2,3-triazol-2-yl)benzamide,
N-{2-[4-(4-fluorophenyl)-1H-pyrazol-1-yl]ethyl}-N,5-dimethyl-2-(2H-1,2,3-triazol-2-yl)benzamide,
N-ethyl-N-{2-[4(5-fluoropyridin-2-yl)-1H-pyrazol-1-yl]ethyl}-5-methyl-2-(2H-1,2,3-triazol-2-yl)benzamide,
N-(cyclopropylmethyl)-N-{2-[4-(5-fluoropyridin-2-yl)-1H-pyrazol-1-yl]ethyl}-5-methyl-2-(2H-1,2,3-triazol-2-yl)benzamide,
N-(cyclopropylmethyl)-N-{2-[4-(4-fluorophenyl)-1H-pyrazol-1-yl]ethyl}-5-methyl-2-(2H-1,2,3-triazol-2-yl)benzamide,
N-ethyl-N-{2-[4-(4-fluorophenyl)-1H-pyrazol-1-yl]ethyl}-5-methyl-2-(2H-1,2,3-triazol-2-yl)benzamide,
N-ethyl-N-{2-[3-(4-fluorophenyl)-1H-pyrazol-1-yl]ethyl}-5-methyl-2-(2H-1,2,3-triazol-2-yl)benzamide,
N-ethyl-5-methyl-N-{2-[3-(pyridin-2-yl)-1H-pyrazol-1-yl]ethyl}-2-(2H-1,2,3-triazol-2-yl)benzamide,
N-{2-[4-(5-chloropyridin-2-yl)-1H-pyrazol-1-yl]ethyl}-N-ethyl-5-methyl-2-(2H-1,2,3-triazol-2-yl)benzamide,
N-ethyl-5-methyl-N-{2-[4-(pyridin-2-yl)-1H-pyrazol-1-yl]ethyl}-2-(2H-1,2,3-triazol-2-yl)benzamide,
N-ethyl-N-{2-[4-(6-methylpyridin-2-yl)-1H-pyrazol-1-yl]ethyl}-5-methyl-2-(2H-1,2,3-triazol-2-yl)benzamide,
N-cyclopropyl-N-{2-[4-(5-fluoropyridin-2-yl)-1H-pyrazol-1-yl]ethyl}-5-methyl-2-(2H-1,2,3-triazol-2-yl)benzamide,
N-{2-[4-(5-fluoropyridin-2-yl)-1H-pyrazol-1-yl]ethyl}-5-methyl-N-(propan-2-yl)-2-(2H-1,2,3-triazol-2-yl)benzamide,
N-ethyl-N-{2-[4-(5-fluoropyridin-2-yl)-1H-pyrazol-1-yl]ethyl}-5-methyl-2-(pyrimidin-2-yl)benzamide,
N-{2-[3-(5-fluoropyridin-2-yl)-1H-pyrazol-1-yl]ethyl}-5-methyl-2-(2H-1,2,3-triazol-2-yl)benzamide,
N-{2-[3-(5-fluoropyridin-2-yl)-1H-pyrazol-1-yl]ethyl}-N,5-dimethyl-2-(2H-1,2,3-triazol-2-yl)benzamide,
N-(cyclopropylmethyl)-N-{2-[3-(5-fluoropyridin-2-yl)-1H-pyrazol-1-yl]ethyl}-5-methyl-2-(2H-1,2,3-triazol-2-yl)benzamide,
N-ethyl-N-{2-[3-(5-fluoropyridin-2-yl)-1H-pyrazol-1-yl]ethyl}-5-methyl-2-(2H-1,2,3-triazol-2-yl)benzamide,
N-{2-[3-(4-fluorophenyl)-1H-pyrazol-1-yl]ethyl}-5-methyl-2-(2H-1,2,3-triazol-2-yl)benzamide,
N-{2-[3-(4-fluorophenyl)-1H-pyrazol-1-yl]ethyl}-N,5-dimethyl-2-(2H-1,2,3-triazol-2-yl)benzamide,
N-{2-[3-(5-fluoropyridin-2-yl)-1H-pyrazol-1-yl]ethyl}-5-methyl-N-(propan-2-yl)-2-(2H-1,2,3-triazol-2-yl)benzamide,
N-(2,2-difluoroethyl)-N-{2-[3-(5-fluoropyridin-2-yl)-1H-pyrazol-1-yl]ethyl}-5-methyl-2-(2H-1,2,3-triazol-2-yl)benzamide,
5-fluoro-N-{2-[3-(5-fluoropyridin-2-yl)-1H-pyrazol-1-yl]ethyl}-2-(2H-1,2,3-triazol-2-yl)benzamide,
N-methyl-N-{2-[3-(5-fluoropyridin-2-yl)-1H-pyrazol-yl]ethyl}-5-fluoro-2-(2H-1,2,3-triazol-2-yl)benzamide,
N-ethyl-N-{2-[3-(5-fluoropyridin-2-yl)-1H-pyrazol-1-yl]ethyl}-5-fluoro-2-(2H,1,2,3-triazol-2-yl)benzamide,
N-{2-[3-(5-fluoropyridin-2-yl)-1H-pyrazol-1-yl]ethyl}-5-methyl-2-(pyrimidin-2-yl)benzamide,
N-ethyl-N-{2-[3-(5-fluoropyridin-2-yl)-1H-pyrazol-1-yl]ethyl}-5-methyl-2-(pyrimidin-2-yl)benzamide,
N-{2-[3-(5-fluoropyridin-2-yl)-1H-pyrazol-1-yl]ethyl}-5-methyl-N-(2-fluoroethyl)-2-(2H-1,2,3-triazol-2-yl)benzamide,
N-(cyclopropylmethyl)-N-{2-[3-(4-fluorophenyl)-1H-pyrazol-1-yl]ethyl}-5-methyl-2-(2H-1,2,3-trizol-2-yl)benzamide,
N-(cyclopropylmethyl)-5-methyl-N-[2-(3-phenyl-1H-pyrazol-1-yl)ethyl]-2-(2H-1,2,3-triazol-2-yl)benzamide,
N-cyclopropyl-N-{2-[3-(5-fluoropyridin-2-yl)-1H-pyrazol-1-yl]ethyl}-5-methyl-2-(2H-1,2,3-triazol-2-yl)benzamide,
N-{2-[3-(5-fluoropyridin-2-yl)-1H-pyrazol-1-yl]ethyl}-5-methyl-N-(oxetan-3-yl)-2-(2H-1,2,3-triazol-2-yl)benzamide,
N-ethyl-N-{2-[3-(5-fluoropyridin-2-yl)-1H-pyrazol-1-yl]ethyl}-1-2-(2H-1,2,3-triazol-2-yl.)benzamide,
5-chloro-N-ethyl-N-{2-[3-(5-fluoropyridin-2-yl)-1H-pyrazol-1-yl]ethyl}-2-(2H-1,2,3-triazol-2-yl)benzamide,
N-ethyl-N-{2-[3-(5-fluoropyridin-2-yl)-1H-pyrazol-1-yl]ethyl}-2-(pyrimidin-2-yl)benzamide,
N-ethyl-N-{2-[3-(5-fluoropyridin-2-yl)-1H-pyrazol-1-yl]ethyl}-5-methyl-2-(pyridin-2-yl)benzamide,
N-ethyl-N-{2-[3-(5-fluoropyridin-2-yl)-1H-1-pyrazol-1-yl]ethyl}-5-methyl-2-(pyridin-3-yl)benzamide,
N-ethyl-N-{2-[4-(5-fluoropyridin-2-yl)-1H-pyrazol-1-yl]ethyl}-2-(pyrimidin-2-yl)benzamide,
5-methyl-N-{2-[3-(6-methylpyridin-2-yl)-1H-pyrazol-1-yl]ethyl}-2-(2H-1,2,3-triazol-2-yl)benzamide,
N-ethyl-5-methyl-N-{2-[3-(6-methylpyridin-2-yl)-1H-pyrazol-1-yl]ethyl}-2-(2H-1,2,3-triazol-2-yl)benzamide,
5-fluoro-N-{2-[3-(5-fluoropyridin-2-yl)-1H-pyrazol-1-yl]ethyl}-2-(pyrimidin-2-yl)benzamide,
N-ethyl-5-fluoro-N-{2-[3-(5-fluoropyridin-2-yl)-1H-pyrazol-1-yl]ethyl}-2-(pyrimidin-2-yl)benzamide,
5-chloro-N-{2-[3-(5-fluoropyridin-2-yl)-1H-pyrazol-1-yl]ethyl}-2-(pyrimidin-2-yl)benzamide,
N-ethyl-5-chloro-N-{2-[3-(5-fluoropyridin-2-yl)-1H-pyrazol-1-yl]ethyl}-2-(pyrimidin-2-yl)benzamide,
N-ethyl-4-fluoro-N-{2-[3-(5-fluoropyridin-2-yl)-1H-pyrazol-1-yl]ethyl}-2-(2H-1,2,3-triazol-2-yl)benzamide,
N-ethyl-2-fluoro-N-{2-[3-(5-fluoropyridin-2-yl)-1H-pyrazol-1-yl]ethyl}-6-(2H-1,2,3-triazol-2-yl)benzamide,
N-ethyl-2-fluoro-N-{2-[3-(5-fluoropyridin-2-yl)-1H-pyrazol-1-yl]ethyl}-6-(pyrimidin-2-yl)benzamide,
N-ethyl-N-{2-[3-(5-fluoropyridin-2-yl)-1H-pyrazol-1-yl]ethyl}-4-methyl-2-(2H-1,2,3-triazol-2-yl)benzamide,
N-ethyl-3-fluoro-N-{2-[3-(5-fluoropyridin-2-yl)-1H-pyrazol-1-yl]ethyl}-2-(2H-1,2,3-triazol-2-yl)benzamide,
N-ethyl-4-fluoro-N-{2-[3-(5-fluoropyridin-2-yl)-1H-pyrazol-1-yl]ethyl}-2-(pyrimidin-2-yl)benzamide,
N-ethyl-N-{2-[3-(5-fluoropyridin-2-yl)-1H-pyrazol-1-yl]ethyl}-5-methoxy-2-(2H-1,2,3-triazol-2-yl)benzamide,
N-ethyl-5-methyl-2-(2H-1,2,3-triazol-2-yl)-N-(2-{3-[5-(trifluoromethyl)pyridin-2-yl]-1H-pyrazol-1-yl}ethyl)benzamide,
N-ethyl-5-methyl-2-(2H-1,2,3-triazol-2-yl)-N-(2-{3-[6-(trifluoromethyl)pyridin-2-yl]-1H-pyrazol-1-yl}ethyl)benzamide,
N-{2-[3-(3,4-difluorophenyl)-1H-pyrazol-1-yl]ethyl}-N-ethyl-5-fluoro-2-(pyrimidin-2-yl)benzamide,
N-ethyl-5-fluoro-N-{2-[3-(4-fluorophenyl)-1H-pyrazol-1-yl]ethyl}-2-(pyrimidin-2-yl)benzamide,
5-chloro-N-ethyl-N-{2-[4-(5-fluoropyridin-2-yl)-1H-pyrazol-1-yl]ethyl}-2-(2H-1,2,3-triazol-2-yl)benzamide,
N-ethyl-5-fluoro-N-{2-[4-(5-fluoropyridin-2-yl)-1H-pyrazol-1-yl]ethyl}-2-(2H-1,2,3-triazol-2-yl)benzamide,
N-ethyl-5-fluoro-2-(pyrimidin-2-yl)-N-(2-{3-[6-(trifluoromethyl)pyridin-2-yl]-1H-pyrazol-1-yl}ethyl)benzamide,
N-ethyl-(pyrimidin-2-yl)-N-(2-{3-[6-(trifluoromethyl)pyridin-2-yl]-1H-pyrazol-1-yl}ethyl)benzamide,
N-{2-[3-(3,4-difluorophenyl)-1H-pyrazol-1-yl]ethyl}-N-ethyl-2-(pyrimidin-1-yl)benzamide,
N-ethyl-5-methyl-2-(2H-1,2,3-triazol-2-yl)-N-(2-{3-[4-(trifluoromethyl)pyridin-2-yl]-1H-pyrazol-1-yl}ethyl)benzamide,
N-ethyl-5-fluoro-2-(pyrimidin-2-yl)-N-(2-{3-[4-(trifluoromethyl)pyridin-2-yl]-1H-pyrazol-1-yl}ethyl)benzamide.

8. A pharmaceutical composition comprising a pyrazole derivative or a pharmaceutically acceptable salt thereof according to any one of claims 1 to 7 as an active ingredient.

9. A therapeutic or prophylactic agent for a disease selected from sleep disorder, depression, anxiety disorder, panic disorder, schizophrenia, drug dependence, Alzheimer's disease, Parkinson's disease, Huntington's chorea, eating disorder, pain, gastrointestinal diseases, epilepsy, inflammation, immune-related diseases, endocrine-related diseases, and hypertension, comprising a pyrazole derivative or a pharmaceutically acceptable salt thereof according to any one of claims 1 to 7 as an active ingredient.
